# EUROPEAN PATENT APPLICATION

(11) **EP 4 209 213 A1**
(43) Date of publication of application: **12.07.2023**
(21) Application number: 22150358.4
(22) Date of filing: 05.01.2022
(51) Int. Cl.: A61K 31/4178, A61K 31/635, A61K 31/444, A61K 31/4545, A61K 31/506, A61P 35/00, A61K 45/06

(54) **SIGNALLING-PATHWAY INHIBITOR COMBINATIONS FOR USE IN THE TREATMENT OF CANCER DISEASES**

(71) Applicant: Universität zu Köln, 50923 Köln (DE)
(72) Inventor: Pasparakis, Manolis, 50933 Köln (DE); Fallais, Simon, 50670 Köln (DE); Knittel, Gero, 50937 Köln (DE); Reinhardt, Christian, 50935 Köln (DE)
(74) Representative: Kuttenkeuler, David

(57) **Abstract**

The present invention pertains to the treatment of proliferative diseases, such as cancer. In particular, the invention describes a novel combination therapy strategy, comprising a Receptor-interacting serine/threonine-protein kinase 1 (RIPK1) inhibitor, an Inhibitor of κB (IκB) Kinase (IKK)/Nuclear Factor κB (NFκB) - signaling inhibitor, and a B-cell lymphoma 2 (BCL-2) inhibitor for the treatment and/or prevention of proliferative diseases, such as cancer. The invention provides such inhibitory compounds and their combinations for use in medical applications, as well as pharmaceutical compositions comprising the compounds of the invention. The invention further pertains to a method of treatment and/or prevention of a proliferative disease in a subject, the method comprising administering to the subject as single treatments or one or more combinatorial treatments, either sequentially or concomitantly, a therapeutically effective amount of inhibitors of RIPK1, inhibitors of IKK/NFκB - signalling, and inhibitors of BCL-2.

## Description

### FIELD OF THE INVENTION

The present invention pertains to the treatment of proliferative diseases, such as cancer. In particular, the invention describes a novel combination therapy strategy, comprising a Receptor-interacting serine/threonine-protein kinase 1 (RIPK1) inhibitor, an Inhibitor of κB (IκB) Kinase (IKK)/Nuclear Factor κB(NFκB) - signaling inhibitor, and a B-cell lymphoma 2 (BCL-2) inhibitor for the treatment and/or prevention of proliferative diseases, such as cancer. The invention provides such inhibitory compounds and their combinations for use in medical applications, as well as pharmaceutical compositions comprising the compounds of the invention. The invention further pertains to a method of treatment and/or prevention of a proliferative disease in a subject, the method comprising administering to the subject as single treatments or one or more combinatorial treatments, either sequentially or concomitantly, a therapeutically effective amount of inhibitors of RIPK1, inhibitors of IKK/NFκB - signalling, and inhibitors of BCL-2.

### DESCRIPTION

Cell death is a necessary biological process for the maintenance of normal tissue homeostasis e.g. by promoting the removal of damaged cells. Cancer cells frequently acquire the ability to block cell death, which contributes to oncogenesis, tumor maintenance and particularly chemo-resistance. Apoptosis is a form of programmed cell death that is involved in tumorigenesis, although other types of regulated cell death that have been more recently discovered, such as necroptosis, are also implicated in cancer.

BCL-2 is a pro-survival protein that has been shown to be dysregulated in multiple lymphoid malignancies, including chronic lymphocytic leukemia, follicular lymphoma, diffuse large B cell lymphoma and others (1, 2). BCL-2 inhibition is thus a promising strategy for the treatment of proliferative diseases, such as cancer, including chronic lymphocytic leukemia, follicular lymphoma, and diffuse large B cell lymphoma.

An FDA-approved BCL-2 inhibitor for the treatment of patients with chronic lymphocytic leukemia (CLL) and small lymphocytic lymphoma (SLL), who have received at least one prior line of therapy, is the compound Venetoclax (also referred to as ABT-199) (3, 4). The clinical use of Venetoclax is currently also being evaluated in patients with other types of Non-Hodgkin lymphoma (3, 4). Venetoclax is also approved as second line treatment in combination with rituximab and approval of venetoclax in combination with obinutuzumab is expected for CLL patients with comorbidities as a first line regimen (5). In NHL cell lines, high BCL-2 expression predicts sensitivity to Venetoclax, which vice-versa is likely to cause treatment limitations in case of relatively low expression of BCL-2 (6).

It was recently shown that oncogenic BRAF mutations, as well as immune escape through CD274 amplification appear to be selected events in CLL patients with acquired venetoclax resistance (7). Moreover, while apoptosis resistance can be achieved by BCL2 upregulation, other proteins can similarly mediate apoptosis resistance. One prominent example in this regard is MCL-1, which is frequently deregulated in neoplastic disease, including various lymphoma entities (8). Thus, venetoclax is a very potent therapeutic agent when used in the right clinical scenario, such as a neoplastic disease with BCL2-mediated apoptosis resistance, or in a setting where venetoclax can mediate apoptosis sensitization as part of a combination regimen.

Particularly aggressive lymphomas of the activated B cell type (ABC-DLBCL) are likely to constitute an entity, where venetoclax may successfully be used. ABC-DLBCL are characterized by two critical features: First, ABC-DLBCL display a high frequency of focal BCL2 amplifications, leading to high BCL2 expression levels (2). Moreover, ABC-DLBCL are characterized by constitutive activation of the pro-survival IKK/NF-kB signaling pathway, which is mediated through recurrent activating mutations in the B cell receptor (BCR) pathway (CD79B, CARD11) and the toll-like receptor (TLR) pathway (MYD88).

However, as with many other targeted agents, acquired resistance against venetoclax limits its clinical use as a single agent. Hence, the present invention seeks to provide novel therapeutic approaches to tackle proliferative diseases, such as cancer, by providing a combined treatment comprising a BCL-2-inhibitor, such as venetoclax. The above problem is solved in a first aspect thereof by combining a BCL-2-inhibitor with an inhibitor of the IKK/NF-kB signaling pathway for use as a treatment of proliferative diseases.

The IKK complex, which is a serine protein kinase and is composed of the regulatory subunit NEMO/IKKγ and the catalytic subunits IKKα and IKKβ, is the major activator of Nuclear factor kappa B (NF-κB) family transcription factors. NF-κB is a ubiquitous transcription factor and an essential mediator of gene expression during activation of immune and inflammatory responses, and a regulator of many anti-apoptotic, and proliferative genes. NF-κB mediates the expression of a great variety of genes in response to extracellular stimuli including IL-1, TNFα, and the bacterial product LPS. NF-κB is associated with IκB proteins in the cell cytoplasm, which inhibit NF-κB activity. IκB kinase (IKK) phosphorylates IκB, and mediates IκB degradation and NF-κB activation. The subunits IKKα and IKKβ interact with each other and both are essential for NF-κB activation, and IKKβ phosphorylates both IκB-alpha and IκB-beta. IKKβ is expressed in variety of human tissues.

NF-kB activity is increased in various tumors, including hematological malignancies (9, 10), and inhibition of NFKB activity kills tumor cells in vitro (11-14). The IκB kinases (IKKs) are key regulatory signaling molecules that coordinate the activation of NF-κB. Many immune and inflammatory mediators, including TNFα, lipopolysaccharide (LPS), IL-Iβ, CD₃/CD28 (antigen presentation), CD40L, FasL, viral infection, and oxidative stress have been shown to lead to NF-κB activation. Although the receptor complexes that transduce these diverse stimuli appear very different in their protein components, it is understood that each of these stimulation events leads to activation of the IKKs and NF-κB. Amongst other mechanisms, NF-kB exerts its pro-survival function by regulating the transcription of anti-apoptotic genes including BCL-2, BCL-xl and BFL-1/A1 (15, 16).

However, despite promising pre-clinical results, safety-concerns have precluded the use of IKK-inhibitors in the clinic. The most severe safety concern due to side effects of IKK inhibitors is the induction of neutrophilia and enhanced inflammation (18). Thus, the use of IKK2 inhibitors in vivo is limited by induced toxicity, e.g. mediated by increased production of IL-1β and the development of neutrophilia. To overcome such limitations, the present invention further seeks to provide an efficient therapeutic treatment for proliferative diseases, which comprises an IKK/NF-κB - signaling inhibitor, while overcoming known adverse effects of such IKK/NF-κB - signaling inhibitor treatments. Thus, the present invention further seeks to provide novel therapeutic approaches to tackle proliferative diseases, such as cancer, by providing a composition and/or combination of compounds having improved activity while being well-tolerated.

The present invention overcomes the above problem by combining a BCL-2 inhibitor and an IKK-inhibitor with a RIPK1-inhibitor. Surprisingly, the inventors found that an increased production of IL-1 beta by myeloid cells treated with IKK inhibitors largely depends on RIPK1 kinase activity. Accordingly, inhibition of RIPK1 kinase activity either genetically (e.g., using knock-in mice expressing kinase-inactive RIPK1 D138N) or pharmacologically (e.g., using Necrostatin-1, a chemical inhibitor of RIPK1) strongly diminishes the LPS-induced production of IL-1β by macrophages treated with IKK inhibitors. In addition, the inventors found that the development of neutrophilia caused by myeloid cell specific inhibition of IKK2 in a mouse model can be prevented by genetic inhibition of RIPK1 kinase activity (e.g., using knock-in mice expressing kinase-inactive RIPK1 D138N, as depicted in Figure 5 of WO 2019/110832). Thus, the most serious known side effects of IKK inhibitors, namely the increased production of IL-1β by myeloid cells and the development of neutrophilia *in vivo,* can be prevented by the inhibition of RIPK1. Hence, the inventors found the surprising effect that the combined use of BCL-2 inhibition and IKK inhibition synergizes to efficiently cause an increased death of cancer cells, and that the simultaneous or concomitant application of RIPK1 inhibitors prevents toxicity induced by IKK inhibition. Thus, inhibition of NF-kB signaling using IKK-inhibitors potentiates pro-apoptotic activity of BCL-2 inhibitors, while inhibition of RIPK1 prevents IKK-inhibition induced toxicity.

### BRIEF DESCRIPTION OF THE INVENTION

Generally, and by way of brief description, the main aspects of the present invention can be described as follows:

In **a first aspect,** the invention pertains to a Receptor-interacting serine/threonine-protein kinase 1 (RIPK1) inhibitor, an Inhibitor of κB (IκB) Kinase (IKK)/Nuclear Factor κB (NFκB) - signaling inhibitor, and a B-cell lymphoma 2 (BCL-2) inhibitor for use in the treatment and/or prevention of a proliferative disease, such as cancer, in a subject.

In **a second aspect,** the invention pertains to a pharmaceutical composition for use in the treatment and/or prevention of a proliferative disease, the pharmaceutical composition comprising a RIPK1 inhibitor, an IKK/ NFκB - signaling inhibitor, and a BCL-2 inhibitor.

In **a third aspect,** the invention pertains to a combination comprising a RIPK1 inhibitor, an IKK/ NFκB - signaling inhibitor, and a BCL-2 inhibitor for use in the treatment or prevention of a proliferative disease, such as cancer, in a subject.

In **a fourth aspect,** the invention pertains to the use of a RIPK1 inhibitor, an IKK/ NFκB - signaling inhibitor, and a BCL-2 inhibitor for the manufacture of a medicament for the treatment and/or prevention of a proliferative disease.

In **a fifth aspect,** the invention pertains to a method of treatment and/or prevention of a proliferative disease in a subject, the method comprising one or more steps of administering to the subject a therapeutically effective amount of a RIPK1 inhibitor, an IKK/ NFκB - signaling inhibitor, and a BCL-2 inhibitor.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the invention pertains to a compound for use in the treatment and/or prevention of a proliferative disease in a subject, wherein the compound is selected from:
**(a)** A Receptor-interacting serine/threonine-protein kinase 1 (RIPK1) inhibitor;
**(b)** An Inhibitor of κB (IκB) Kinase (IKK)/Nuclear Factor κB (NFκB) - signaling inhibitor; and
**(c)** A B-cell lymphoma 2 (BCL-2) inhibitor,
wherein if the compound is (a), the subject will receive, receives or has received an additional treatment with (b) and (c), or if the compound is (b), the subject will receive, receives or has received an additional treatment with (a) and (c), or if the compound is (c), the subject will receive, receives or has received an additional treatment with (a) and (b).

Without being bound to theory, the present invention is based on two surprising effects: First, BCL-2 inhibition and IKK inhibition synergize to cause increased death of cancer cells, because inhibition of NF-kB signaling using IKK-inhibitors potentiates pro-apoptotic activity of BCL-2 inhibitors. However, IKK inhibitors have strong side effects, such as toxicity mediated by increased production of IL-1 beta, leading to the development of neutrophilia. Since an increased production of IL-1 beta by myeloid cells treated with IKK inhibitors depends largely on RIPK1 kinase activity, inhibition of RIPK1 kinase activity either genetically (e.g., using knock-in mice expressing kinase-inactive RIPK1 D138N) or pharmacologically (e.g., using Necrostatin-1, a chemical inhibitor of RIPK1) strongly diminishes the LPS-induced production of IL-1β by macrophages treated with IKK inhibitors. Moreover, genetic inhibition of RIPK1 kinase activity (e.g., using knock-in mice expressing kinase-inactive RIPK1 D138N) prevents the development of neutrophilia caused by myeloid cell-specific inhibition of IKK2. This demonstrates that the most serious known side effect of IKK inhibitors, namely the increased production of IL-1β by myeloid cells, can be prevented by the inhibition of RIPK1.

Thus, the second surprising effect of this invention relates to preventing toxicity caused by the use of an IKK inhibitor. In cancer therapy, striking a good balance between efficacy and tolerability is of particular importance, and there is evidence that the clinical use of IKK inhibitors has so far been hindered by safety-concerns. Hence, the invention is based on the surprising effect of a combined use of BCL-2 inhibition and IKK inhibition to synergize in causing increased death of cancer cells, and on preventing IKK inhibition induced toxicity by RIPK1 inhibition.

The term "inhibitor of Receptor-interacting serine/threonine-protein kinase 1 (RIPK1)", "RIPK1 inhibitor" or "inhibitor of RIPK1" or "antagonist of RIPK1" or any similar expressions shall in context of the present invention encompass any compound or combination of compounds that have an activity as a modulator of the expression, function and/or stability of RIPK1, or of a variant of RIPK1.

The term "inhibitor of κB (IκB) Kinase (IKK)/Nuclear Factor κB (NFκB) - signaling", "inhibitor of IKK/NFκB" or "IKK/NFκB inhibitor" or "antagonist of IKK/NFκB" or any similar expressions shall in context of the present invention encompass any compound or combination of compounds that have an activity as a modulator of the expression, function and/or stability of IKK/NFκB, or of a variant of IKK/NFκB.

The term "inhibitor of B-cell lymphoma 2 (BCL-2)", "BCL-2 inhibitor" or "inhibitor of BCL-2" or "antagonist of BCL-2" or any similar expressions shall in context of the present invention encompass any compound or combination of compounds that have an activity as a modulator of the expression, function and/or stability of BCL-2, or of a variant of BCL-2.

In context of the invention a modulator is preferably an inhibitor/antagonist.

Further, in context of the present invention the term "receptor interacting serine/threonine kinase 1" or "RIPK1" pertains to a human gene encoding for a protein according to the amino acid sequence shown in SEQ ID NO: 1. RIPK1 is also known as "receptor (TNFRSF)-interacting serine-threonine kinase 1" or "receptor-interacting protein kinase 1" (RIP) (HUGO Gene Nomenclature Committee symbol: HGNC:10019, database version of November 2017). The human RIPK1 gene is located 6p25.2, homologs are known from mouse (MGI:108212; NCBI Gene: 19766) and rat (Rat Genome Database (RGD) ID: 1310158).

The terms "RIPKi-protein" or "protein of RIPK1" as used in context of the herein disclosed invention shall pertain to a protein (such as a full-length protein, fusion protein or partial protein) comprising a sequence as shown in SEQ ID NO: 1. The terms shall also refer to a protein comprising the amino acid sequence according to SEQ ID NO: 1 with any protein modifications. Such protein modifications preferably do not alter the amino acid sequence of the polypeptide chain, but constitute a functional group, which is conjugated to the basic amino acid polymer chain. Protein modifications in context of the invention may be selected from a conjugation of additional amino acid sequences to the RIPK1 amino acid chain, such as ubiquitination, sumoylation, methylation, or similar small protein conjugates. Other protein modifications include, but are not limited to, glycosylation, methylation, lipid-conjugation, or other natural or artificial post-translational modifications known to the skilled person. The terms "protein of a variant of RIPK1" and the like, shall have the corresponding meaning with respect to a variant of RIPK1.

The terms "RIPKi-mRNA" or "mRNA of RIPK1" as used in context of the herein disclosed invention shall pertain to a messenger ribonucleic acid (such as a full-length mRNA, fusion mRNA or partial mRNA, and/or splice-variants thereof) comprising a region encoding for an amino acid sequence as shown in SEQ ID NO: 1. The terms shall also refer to an mRNA comprising a region encoding for the amino acid sequence according to SEQ ID NO: 1 with any codon or nucleotide modifications. Such modifications preferably would not alter the amino acid sequence of the encoded polypeptide chain. The terms "mRNA of a variant of RIPK1" and the like, shall have the corresponding meaning with respect to a variant of RIPK1.

A variant of RIPK1 is, in some embodiments, a protein comprising an amino acid sequence having at least 60%, 70%, 80%, 90%, preferably at least 80% such as at least 90%, sequence identity to SEQ ID NO: 1, and most preferably at least 95% (such as at least 98%) sequence identity to SEQ ID NO: 1 (the human RIPK1 amino acid sequence). In one preferred embodiment of the invention, the variant of RIPK1 comprises an amino acid sequence with at least 80% sequence identity to the amino acid sequence shown in SEQ ID NO: 1.

As used herein, the terms "identical" or percent "identity", when used anywhere herein in the context of two or more nucleic acid or protein/polypeptide sequences, refer to two or more sequences or subsequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using a sequence comparison algorithms, or by manual alignment and visual inspection (see, e.g., NCBI web site).

Preferred RIPK1 inhibitors of the invention are small molecular compounds. Such compounds are preferably selected from necrostatin-1 stable (5-((7-chloro-1 H-indol-3-yl)methyl)-3-methyl-2,4-imidazolidinedione), necrostatin-1(5-(1H-indo1-3-ylmethyl)-3-methyl-2-thioxo-4-imidazolidinone), necrostatin-2(5-[(7-chloro-1H-indol-3-yl)methyl]-3-methyl-2,4-imidazolidinedione), necrostatin-3(3-phenyl-3,3a,4,5-tetrahydro-2H-benz[g]indazole, 3-phenyl-2,3,3a,4-tetrahydro[1]benzopyrano[4,3-c]pyrazole, 3-phenyl-2,3,3a,4-tetrahydro[1]benzothiopyrano[4,3-c]pyrazole, 5,5-dioxo-3-phenyl-2,3,3a,4-tetrahydro[1]benzothiopyrano[4,3-c]pyrazole]), necrostatin-4((S)-N-(1-[2-chloro-6-fluorophenyl]ethyl)-5-cyano-1-methyl-1H-pyrrole-2-carboxamide), necrostatin-5(2-[[3,4,5,6,7,8-hexahydro-3-(4-methoxyphenyl)-4-oxo[1]benzothieno[2,3-d]pyrimidin-2-yl]thio]-acetonitrile), necrostatin-7(5-((3-(4-fluorophenyl)-1H-pyrazol-4-yl)methylene)-2-imino-3-(thiazol-2-yl)thiazolidin-4-one), necrostatin-21, (S)-5-benzyl-N-(7,9difluoro-2-oxo-2,3,4,5-tetrahydro-1H-benzo[b]azepin-3-yl)1H-1,2,4-triazole-3-carboxamide (GSK3145095), N-benzyl-N-hydroxy-2,2-dimethylbutanamide (RIPA-56), DNL747 (SAR 443060), (*S*)-*N*-(5-methyl-4-oxo-2,3,4,5-tetrahydrobenzo[*b*][1,4]oxazepin-3-yl)-4,5,6,7-tetrahydro-1*H*-indazole-3-carboxamide, (*S*)-*N*-(5-methyl-4-oxo-2,3,4,5-tetrahydrobenzo[*b*][1,4]oxazepin-3-yl)-4*H*-1,2,4-triazole-3-carboxamide, (*R*)-5-methyl-*N*-((*S*)-5-methyl-4-oxo-2,3,4,5-tetrahydrobenzo[*b*][1,4]oxazepin-3-yl)-4,5,6,7-tetrahydro-1H-indazole-3-carboxamide, (*R*)-5-methyl-*N*-((S)-5-methyl-4-oxo-2,3,4,5-tetrahydrobenzo[*b*][1,4]oxazepin-3-yl)-4,5,6,7-tetrahydro-1H-indazole-3-carboxamide, (S)-6,6-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (S)-N-(5-methyl-4-oxo-2,3,4,5-tetrahydrobenzo[b][1,4]oxazepin-3-yl)-1',4',5',7'-tetrahydrospiro[cyclopropane-1,6'-indazole]-3'-carboxamide, N-((S)-5-methyl-4-oxo-2,3,4,5-tetrahydrobenzo[b][1,4]oxazepin-3-yl)-1,4,4a,5,5a,6-hexahydrocyclopropa[flindazole-3-carboxamide, N-((S)-5-methyl-4-oxo-2,3,4,5-tetrahydrobenzo[b][1,4]oxazepin-3-yl)-3,4,5,5a,6,6a-hexahydrocyclopropa[e]indazole-1-carboxamide, (S)-5,5-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (R)-5-(tert-butyl)-N-((S)-5-methyl-4-oxo-2,3,4,5-tetrahydrobenzo[b][1,4]oxazepin-3-yl)-4,5,6,7-tetrahydro-1H-indazole-3-carboxamide, N-((S)-5-methyl-4-oxo-2,3,4,5-tetrahydrobenzo[b][1,4]oxazepin-3-yl)-5-phenyl-4,5,6,7- tetrahydro-1H-indazole-3-carboxamide, N-((S)-5-methyl-4-oxo-2,3,4,5-tetrahydrobenzo[b][1,4]oxazepin-3-yl)-5-(1H-pyrazol-1-yl)-4,5,6,7-tetrahydro-1H-indazole-3-carboxamide, (R)-1-methyl-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (S)-N-(5-methyl-4-oxo-2,3,4,5-tetrahydrobenzo[b][1,4]oxazepin-3-yl)-5-(trifluoromethyl)-1H-indazole-3-carboxamide, (S)-N-(5-methyl-4-oxo-2,3,4,5-tetrahydrobenzo[b][1,4]oxazepin-3-yl)-5-(trifluoromethyl)-1H-pyrazolo[3,4-b]pyridine-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-[1,2,3]triazolo [1,5-a]pyridine-3-carboxamide, (S)-N-(5-methyl-4-oxo-2,3,4,5-tetrahydrobenzo[b][1,4]oxazepin-3-yl)-7-(trifluoromethyl)imidazo[1,5-a]pyridine-1-carboxamide, (S)-N-(5-methyl-4-oxo-2,3,4,5-tetrahydrobenzo[b][1,4]oxazepin-3-yl)-6-(trifluoromethyl)-[1,2,4]triazolo[4,3-b]pyridazine-3-carboxamide, (S)-5-isopropyl-N-(5-methyl-4-oxo-2,3,4,5-tetrahydrobenzo[b][1,4]oxazepin-3-yl)-1H-pyrazolo[3,4-b]pyridine-3-carboxamide, (S)-N-(5-methyl-4-oxo-2,3,4,5-tetrahydrobenzo[b][1,4]oxazepin-3-yl)-5-(perfluoroethyl)-1H-pyrazolo[3,4-b]pyridine-3-carboxamide, N-((S)-5-methyl-4-oxo-2,3,4,5-tetrahydrobenzo[b][1,4]oxazepin-3-yl)-5-(perfluoroethyl)-4,5,6,7-tetrahydro-[1,2,3]triazolo[1,5-a]pyridine-3-carboxamide, (S)-N-(5-methyl-4-oxo-2,3,4,5-tetrahydrobenzo[b][1,4]oxazepin-3-yl)-6-(perfluoroethyl)-[1,2,4]triazolo[4,3-b]pyridazine-3-carboxamide, (S)-5-benzyl-N-(5-methyl-4-oxo-2,3,4,5-tetrahydrobenzo[b][1,4]oxazepin-3-yl)isoxazole-3-carboxamide (GSK481), 1-[(5S)-4,5-Dihydro-5-phenyl-1H-pyrazol-1-yl]-2,2-dimethyl-1-propanone, 2,2-Dimethyl-1-(5(S)-phenyl-4,5-dihydro-pyrazol-1-yl)-propan-1-one (GSK963), (S)-5-benzyl-N-(5-methyl-4-oxo-2,3,4,5-tetrahydrobenzo[b][1,4]oxazepin-3-yl)-1H-1,2,4-triazole-3-carboxamide (GSK2982772), 5-(indol-3-ylmethyl)-3-methyl-2-thio-hydantoin), and their derivatives or analogues. In a particularly preferred embodiment, the RIPK1 inhibitor is necrostatin-1 stable (Nec-is). However, any other known RIPK1 inhibitor may be used in context of the invention.

Further preferred RIPK1 inhibitors of the invention are small molecular compounds as disclosed in WO 2017/136727. In particular, a RIPK1 inhibitor useful in context of the present invention may be a compound selected from those compounds in Table 1, 2, 3 or 4 in WO 2017/136727 (such compounds of tables 1, 2, 3 and 4 in WO 2017/136727 are incorporated herein by reference), or stereoisomers and mixtures of stereoisomers thereof. Also included within the disclosure is a compound selected from Table 1, 2, 3 or 4 in WO 2017/136727, as a pharmaceutically acceptable salt thereof.

Further preferred RIPK1 inhibitors of the invention are small molecular compounds as disclosed in WO 2018/109097. In particular, a RIPK1 inhibitor useful in context of the present invention may be a compound selected from those compounds as listed beginning on page 4 line 3 and ending on page 6 line 25 of WO 2018/109097 - the listing of compounds is incorporated herein by reference.

Particularly preferred in the context of the present invention is an IKK/NFκB signaling inhibitor (b), which is an IKK inhibitor, preferably an IKK2/IKKβ inhibitor. The protein is known as IKK-beta, IKK2, IKKB, IKBKB, IKKB, IMD15, IMD15A, IMD15B, NFKBIKB, IKKβ, or "inhibitor of nuclear factor kappa B kinase subunit beta" (Human Gene Nomenclature Committee symbol HGNC:5960) and is a protein comprising the amino acid sequence shown in SEQ ID NO: 2 (see https://www.genenames.org/). In context of the present invention the term "inhibitor of IKK2" or "inhibitor of IKKβ" shall pertain to a human gene encoding for a protein according to the amino acid sequence shown in SEQ ID NO: 2 The human IKK2 gene is located at 8p11.21 (NCBI Gene: 3551). The IKK2/IKK-β protein phosphorylates the inhibitor in the inhibitor/NF-kappa-B complex, causing dissociation of the inhibitor and activation of NF-kappa-B. The encoded protein itself is found in a complex of proteins. Several transcript variants have been found for this gene, some of which are protein-coding and some of which are not protein-coding.

The terms "IKK2 protein", "protein of IKK2", "IKK-β protein" or "protein of IKK-β", as used in context of the herein disclosed invention shall pertain to a protein (such as a full-length protein, fusion protein or partial protein) comprising a sequence as shown in SEQ ID NO: 2. The terms shall also refer to a protein comprising the amino acid sequence according to SEQ ID NO: 2 with any protein modifications. Such protein modifications preferably do not alter the amino acid sequence of the polypeptide chain, but constitute a functional group, which is conjugated to the basic amino acid polymer chain. Protein modifications in context of the invention may be selected from a conjugation of additional amino acid sequences to the IKK amino acid chain, such as ubiquitination, sumoylation, methylation, or similar small protein conjugates. Other protein modifications include, but are not limited to, glycosylation, methylation, lipid-conjugation, or other natural or artificial post-translational modifications known to the skilled person. The terms "protein of a variant of IKK" and the like, shall have the corresponding meaning with respect to a variant of IKK.

The terms "IKK2-mRNA", "mRNA of IKK2", "IKK-β-mRNA" or "mRNA of IKK-β", as used in context of the herein disclosed invention shall pertain to a messenger ribonucleic acid (such as a full-length mRNA, fusion mRNA or partial mRNA, and/or splice-variants thereof) comprising a region encoding for an amino acid sequence as shown in SEQ ID NO: 2. The terms shall also refer to an mRNA comprising a region encoding for the amino acid sequence according to SEQ ID NO: 2 with any codon or nucleotide modifications. Such modifications preferably would not alter the amino acid sequence of the encoded polypeptide chain. The terms "mRNA of a variant of IKK" and the like, shall have the corresponding meaning with respect to a variant of IKK, such as of IKK2/IKK-β.

A variant of IKK2/IKK-β is, in some embodiments, a protein comprising an amino acid sequence having at least 60%, 70%, 80%, 90%, preferably at least 80% such as at least 90%, sequence identity to SEQ ID NO: 2, and most preferably at least 95% (such as at least 98%) sequence identity to SEQ ID NO: 2 (the human IKK2/IKK-β amino acid sequence). In one preferred embodiment of the invention, the variant of IKK2/IKK-β comprises an amino acid sequence with at least 80% sequence identity to the amino acid sequence shown in SEQ ID NO: 2.

The term "IKK inhibitor" is used in the broadest sense and includes any molecule that partially or fully blocks, inhibits or neutralizes a biological activity mediated by IKK, preferably by preventing the activation of IKK. The aforesaid with regard to the nature and definition of inhibitors of IKK in context of the invention shall equally apply to the nature and definition of "IKK inhibitors". In addition thereto, preferred IKK inhibitors act directly on one or more subunits of IKK for example by binding to one or more subunits of IKK. However, in other embodiments the IKK inhibitors may prevent IKK from interacting with a substrate, such as I-κB and/or may act on molecules in an IKK signaling pathway, preferably downstream from IKK. In still other embodiments the IKK inhibitors may modulate the level of IKK gene expression or otherwise reduce the levels of IKK in affected cells. The ability of a molecule to inhibit IKK activation can be measured using assays that are well known in the art. For example and without limitation, IKK inhibitors can be identified using immune complex kinase assays and gene reporter assays. Briefly, in an immune complex kinase assay, immunoprecipitated IKK complexes are examined for the ability to phosphorylate GST-IκBα in vitro. For example, IKK complexes can be immunoprecipitated from cleared striatal extracts from animals or cells treated with the putative IKK inhibitor by incubation with a mouse anti-IKKα antibody (Santa Cruz Biotechnology) coupled to protein-A beads and rocked for 3 hr at 4° C. Beads are washed, and IKK activity can be evaluated in vitro with 1 µg of purified GST-Iκ-Bα (N-terminal 61 amino acids) in the presence of 10 µCi of [32P]γ-ATP for 30 min at 30° C. Products are examined by SDS-PAGE followed by autoradiography.

Furthermore, the term "IKK inhibitor" includes any molecule that mimics a biological activity mediated by an IKK subunit and specifically changes the function or expression of IKK, or the efficiency of signaling through IKK, thereby inhibiting an already existing biological activity or triggering a new biological activity.

Preferred IKK inhibitors of the invention are IKK2/IKKβ inhibitors, which are selected from the group consisting of N-(6-chloro-7-methoxy-9H-beta-carbolin-8-yl)-2-methylnicotinamide (ML120B), LY2409881, (3S)-N-(6-Chloro-9H-beta-carbolin-8-yl)-4-(2-((2R,6S)-2,6-dimethylmorpholin-4 - yl)-2-oxoethyl)-6,6-dimethylmorpholine-3-carboxamide (MLN-0415), 2-(pyrimidin-4-yl)-1H-indole (SAR-113945), 1-(5-Methoxy-2-thiophen-2-yl-quinazolin-4-ylamino)-3-methyl-pyrrole-2,5-dione (CDC-839), 1H-imidazo[4,5-b]pyridin-5-amine, 7-[5-[(cyclohexylmethylamino)-methyl]-1H-indol-2-yl]-2-methyl, sulfate trihydrate (E-6070), TPCA-1, Bardoxolone Methyl, Resveratrol, MRT67307, AmLexanox, BMS-345541, IKK 16, BI605906, ACHP Hydrochloride, PS-1145, Apigenin, SC-514, IKKε-IN-1, IMD-0354, AZD 3264, Icariin (Ieariline), Bay 65-1942 Hcl, Bay 65-1942, an Anilino-Pyrimidine, IKK inhibitor III, SC-514*Amino-imidazolecarboxamide, an Ureudo-thiophenecarboxamide, a Diarylpybidine, a Pyridooxazinone, an Indolecarboxamide, a Benzoimidazole carboxamide, a Pyrazolo[4,3-c]quinoline, an Imidazolylquinoline-carbxaldehyde semicarbazide, a Pyridyl Cyanoguanidine, IkB Kinase Inhibitor Peptide, IKK-2 Inhibitor IV ([5-(p-Fluorophenyl)-2-ureido]thiophene-3-carboxamide), IKK Inhibitor II, Wedelolactone, IKK Inhibitor VII, IKK-2 Inhibitor V (N-(3,5-Bis-trifluoromethylphenyl)-5-chloro-2-hydroxybenzamide), IKK-2 Inhibitor VI (5-Phenyl-2-ureido)thiophene-3-carboxamide), IKK-2 Inhibitor VIII ACHP (2-Amino-6-(2-(cyclopropylmethoxy)-6-hydroxyphenyl)-4-(4-piperidinyl)-3-pyridinecarbonitrile), and their derivatives or analogues. In a particularly preferred embodiment, the IKK inhibitor is ML120B or LY2409881. However, any other known IKK inhibitor may be used in context of the invention.

Further, in context of the present invention, the term "B-cell lymphoma 2" or "Bcl-2" pertains to a human gene encoding for a protein according to the amino acid sequence shown in SEQ ID NO: 3. The human Bcl-2 gene is located at 18q21.33 (NCBI Gene: 596). Bcl-2 is also known as PPP1R50. The Bcl-2 gene encodes an integral outer mitochondrial membrane protein that blocks the apoptotic death of cells such as lymphocytes. Constitutive expression of BCL2, such as in the case of translocation of BCL2 to Ig heavy chain locus, is thought to be the cause of follicular lymphoma. Alternative splicing results in multiple transcript variants of BCL2.

The terms "BCL-2-protein" or "protein of BCL-2" as used in context of the herein disclosed invention shall pertain to a protein (such as a full-length protein, fusion protein or partial protein) comprising a sequence as shown in SEQ ID NO: 3. The terms shall also refer to a protein comprising the amino acid sequence according to SEQ ID NO: 3 with any protein modifications. Such protein modifications preferably do not alter the amino acid sequence of the polypeptide chain, but constitute a functional group, which is conjugated to the basic amino acid polymer chain. Protein modifications in context of the invention may be selected from a conjugation of additional amino acid sequences to the BCL-2 amino acid chain, such as ubiquitination, sumoylation, methylation, or similar small protein conjugates. Other protein modifications include, but are not limited to, glycosylation, methylation, lipid-conjugation, or other natural or artificial post-translational modifications known to the skilled person. The terms "protein of a variant of BCL-2" and the like, shall have the corresponding meaning with respect to a variant of BCL-2.

The terms "BCL-2-mRNA" or "mRNA of BCL-2" as used in context of the herein disclosed invention shall pertain to a messenger ribonucleic acid (such as a full-length mRNA, fusion mRNA or partial mRNA, and/or splice-variants thereof) comprising a region encoding for an amino acid sequence as shown in SEQ ID NO: 3. The terms shall also refer to an mRNA comprising a region encoding for the amino acid sequence according to SEQ ID NO: 3 with any codon or nucleotide modifications. Such modifications preferably would not alter the amino acid sequence of the encoded polypeptide chain. The terms "mRNA of a variant of BCL-2" and the like, shall have the corresponding meaning with respect to a variant of BCL-2.

A variant of BCL-2 is, in some embodiments, a protein comprising an amino acid sequence having at least 60%, 70%, 80%, 90%, preferably at least 80% such as at least 90%, sequence identity to SEQ ID NO: 3, and most preferably at least 95% (such as at least 98%) sequence identity to SEQ ID NO: 3 (the human BCL-2 amino acid sequence). In one preferred embodiment of the invention, the variant of BCL-2 comprises an amino acid sequence with at least 80% sequence identity to the amino acid sequence shown in SEQ ID NO:3.

Preferred BCL-2 inhibitors of the present invention are selected from the group consisting of 4-[4-[[2-(4-chlorophenyl)-5,5-dimethyl-1-cyclohexen-1-yl]methyl]-1-piperazinyl]-N-[[4-[[(1R)-3-(4-morpholinyl)-1-[(phenylthio)methyl]propyl]amino]-3-[(trifluoromethyl)sulfonyl]phenyl] sulfonyl]benzamide (navitoclax or ABT-263), tetrocarcin A, antimycin, gossypol (such as (-)-gossypol acetic acid), obatoclax (GX15-070), ethyl 2-amino-6-bromo-4-(1-cyano-2-ethoxy-2-oxoethyl)-4H-chromene-3-carboxylate (HA 14-1), oblimersen, a Bak BH3 peptide, 4-[4-[(4'-chloro[1,1'-biphenyl]-2-yl)methyl]-1-piperazinyl]-N-[[4-[[(1R)-3-(dimethylamino)-1-[(phenylthio)methyl]propyl]amino]-3-nitrophenyl]sulfonyl]-benzamide (ABT-737), 2-((1H-pyrrolo[2,3-b]pyridin-5-yl)oxy)-4-(44(4'-chloro-5,5-dimethyl-3,4,5,6-tetrahydro-[1,1'-biphenyl]-2-Amethyl)piperazin-1-yl)-N-((3-nitro -4-(((tetrahydro-2H-pyran-4-Amethyl)amino)phenyl)sulfonyl)benzamide (venetoclax), S55746 (BCL201), and their derivatives or analogues. In a particularly preferred embodiment, the BCL-2 inhibitor is venetoclax. However, any other known BCL-2 inhibitor may be used in context of the invention.

Venetoclax is a BH3-mimetic, which blocks the anti-apoptotic B-cell lymphoma-2 (BCL-2) protein, leading to programmed cell death of chronic lymphocytic leukemia (CLL) cells. Venetoclax is also known as ABT-199, RG7601, and GDC-0199.

A "modulator of expression, function and/or stability of RIPK1, or of a variant of RIPKi", a "modulator of expression, function and/or stability of IKK/NFκB - signaling inhibitor, or of a variant of IKK/NFκB - signalling", a "modulator of expression, function and/or stability of BCL-2, or of a variant of BCL-2", or grammatically similar expressions, in context of the invention may be any compound that affects, for example in case of an inhibitor/antagonist impairs or interferes with, the expression, function and/or stability of RIPK1, IKK/NFκB - signalling, or BCL-2 respectively, or of a variant of these targets, in particular the expression, function and/or stability of a protein of RIPK1, IKK/NFκB - signalling, or BCL-2 or their variant, and/or the expression, function and/or stability of mRNA of RIPK1, IKK/NFκB - signalling, or BCL-2 or their variant.

In a further preferred embodiment of the present invention, any one of the RIPK1 inhibitor (a), the IKK/NFκB - signaling inhibitor (b), and the BCL-2 inhibitor (c) is selected from a small molecule, a polypeptide, peptide, glycoprotein, a peptide-mimetic, an antigen binding protein (ABP) (for example, an antibody, antibody-like molecule or other antigen binding derivative, or an or antigen binding fragment thereof), a nucleic acid such as a DNA or RNA, for example an antisense or inhibitory DNA or RNA, a ribozyme, an RNA or DNA aptamer, RNAi, siRNA, shRNA and the like, including variants or derivatives thereof such as a peptide nucleic acid (PNA), a genetic construct for targeted gene editing, such as a CRISPR/Cas9 construct and/or a guide nucleic acid (gRNA or gDNA) and/or tracrRNA.

In another preferred embodiment, the modulator of expression, function and/or stability of RIPK1, IKK/NFκB - signalling, or BCL-2, or of a variant of RIPK1, IKK/NFκB - signalling, or BCL-2, is an anti-sense nucleotide molecule such as described in detail herein below, more preferably one that binds to, such as specifically binds to, a nucleic acid that encodes or regulates the expression of RIPK1, IKK/NFκB - signalling, or BCL-2, or of a variant of RIPK1, IKK/NFκB - signalling, or BCL-2, or more preferably one that binds to, such as specifically bind to, a nucleic acid that encodes (or regulates the expression of a gene that controls the expression, function and/or stability of) RIPK1, IKK/NFκB - signalling, or BCL-2, or of a variant of RIPK1, IKK/NFκB - signalling, or BCL-2.

As used herein, the terms "inhibitor of RIPK1 expression", "inhibitor of IKK/NFκB - signaling expression", "inhibitor of BCL-2 expression", and the like (including similarly, "antagonist of RIPK1 expression", "antagonist of IKK/NFκB - signaling expression", "antagonist of BCL-2 expression" and the like) shall relate to any of the herein disclosed modulators (for example, the antigen binding constructs or anti-sense molecules described herein), which has an antagonistic activity toward the expression of an RIPK1, IKK/NFκB - signalling, or BCL-2 protein, such that it impairs, suppresses, reduces and/or lowers the expression of an RIPK1, IKK/NFκB - signalling, or BCL-2 protein such as may be determined by measuring an amount (or change in an amount) of RIPK1, IKK/NFκB - signalling, or BCL-2 protein or RIPK1, IKK/NFκB - signalling, or BCL-2 mRNA. The term "expression" means in this context the cellular process of transcribing a gene into an mRNA and the following translation of the mRNA into a protein. "Gene expression" therefore may refer only to the generation of mRNA, irrespectively from the fate of the so produced mRNA, or alternatively/additionally to the translation of the expressed mRNA into a protein. The term "protein expression" on the other hand shall refer to the complete cellular process of synthesis of proteins. In one preferred example, an inhibiting modulator of the invention, such as an anti-sense molecule, may bind to the RIPK1, IKK/NFκB - signalling, or BCL-2 gene or mRNA and reduce transcription and/or translation of the RIPK1, IKK/NFκB - signalling, or BCL-2 mRNA. The terms "inhibitor of expression of a variant of RIPK1, IKK/NFκB - signalling, or BCL-2" and the like, shall have the corresponding meaning with respect to a variant of RIPK1, IKK/NFκB - signalling, or BCL-2.

The terms "inhibitor of RIPK1 stability", "inhibitor of IKK/NFκB - signaling stability", "inhibitor of BCL-2 stability" and the like (including similarly, "antagonist of RIPK1 stability", "antagonist of IKK/NFκB - signaling stability", "antagonist of BCL-2 stability" and the like) shall refer to any of the herein disclosed modulators (for example, the antigen binding constructs or anti-sense molecules described herein), which has a negative activity towards the stability of an RIPK1, IKK/NFκB - signalling, or BCL-2 protein.

The terms an "inhibitor of RIPK1 function", "inhibitor of IKK/NFκB - signaling function", "inhibitor of BCL-2 function", and the like (including similarly, "antagonist of RIPK1 function", "antagonist of IKK/NFκB - signaling function", "antagonist of BCL-2 function" and the like) shall refer to any of the herein disclosed modulators (for example, the antigen binding constructs or anti-sense molecules described herein) that impairs, such as induces a decrease or reduction in the amount or rate of one or more activities of RIPK1, IKK/NFκB - signaling, or BCL-2 protein or mRNA (for example, by impairing the expression and/or stability of RIPK1, IKK/NFκB - signaling, or BCL-2 protein or mRNA), such as one or more of those activities described herein, for example, the kinase activity of RIPK1, IKK/NFκB - signaling, or BCL-2.

Such an inhibiting modulator can act directly, for example, by binding to a RIPK1, IKK/NFκB - signaling, or BCL-2 protein and decreasing the amount or rate of one or more of the properties of RIPK1, IKK/NFκB - signaling, or BCL-2 such as its expression, function and/or stability. An RIPK1, IKK/NFκB - signaling, or BCL-2 antagonist or inhibitor can also decrease the amount or rate of a RIPK1, IKK/NFκB - signaling, or BCL-2 protein function or activity by impairing its expression, stability, for example, by binding to a RIPK1, IKK/NFκB - signaling, or BCL-2 protein or mRNA and modifying it, such as by removal or addition of a moiety, or altering its three-dimensional conformation; and by binding to a RIPK1, IKK/NFκB - signaling, or BCL-2 protein or mRNA and reducing its stability or conformational integrity. An RIPK1, IKK/NFκB - signaling, or BCL-2 antagonist or inhibitor can also act indirectly, for example, by binding to a regulatory molecule or gene region to modulate regulatory protein or gene region function and affect a decrease in the amount or rate of a RIPK1, IKK/NFκB - signaling, or BCL-2 expression, function and/or stability, in particular by impairing the activity of one or more of a RIPK1, IKK/NFκB - signaling, or BCL-2 protein or mRNA (such as by changing the amount or rate of expression and/or stability of RIPK1, IKK/NFκB - signaling, or BCL-2 protein or mRNA). Thus, an RIPK1, IKK/NFκB - signaling, or BCL-2 inhibitor or antagonist can act by any mechanisms that impair, such as result in a decrease in, the amount or rate of RIPK1, IKK/NFκB - signaling, or BCL-2 expression, function and/or stability.

In some preferred embodiments it might be preferred that the RIPK1, IKK/NFκB - signaling, or BCL-2 inhibitor is a compound that only affects and impairs the enzymatic activity or function of RIPK1, IKK/NFκB - signaling inhibitor, or BCL-2 without interfering with RIPK1, IKK/NFκB - signaling, or BCL-2 protein expression or protein stability. The term "kinase activity" as used herein refers to a phosphorylation of a substrate including RIPK1, IKK/NFκB - signaling, or BCL-2 by RIPK1, IKK/NFκB - signaling, or BCL-2 protein. Such embodiments might be advantageous, because the impairment of RIPK1, IKK/NFκB - signaling, or BCL-2 could induce or cause additional undesirable adverse effects in a treated subject or patient. In order to specifically target and reduce/inhibit RIPK1, IKK/NFκB - signaling, or BCL-2 enzymatic activity, the present invention in some preferred embodiments pertains to compounds which selectively bind to and impair enzymatic activity, such as small molecular compounds, for example kinase inhibitors.

The above problem is further solved by providing a compound for use in the prevention and/or treatment of a proliferative disease according to the present invention, wherein the prevention and/or treatment comprises the concomitant or sequential administration of the RIPK1 inhibitor, IKK/NFκB - signaling inhibitor, and BCL-2 inhibitor as defined herein. Hence, the present invention provides the combined use of a RIPK1 inhibitor, an IKK/NFκB signaling inhibitor, such as an IKK2/IKKβ inhibitor, and a BCL-2 inhibitor, in the treatment of a subject suffering from a proliferative disease, such as cancer. Surprisingly, the combination of compounds of the invention overcomes the problems of the prior art by achieving a potentiation of the pro-apoptotic activity of BCL-2 inhibition by IKK-inhibition, while simultaneously preventing IKK-inhibition-induced toxicity by RIPK1 inhibition.

A particularly preferred embodiment relates to the compound for use according to the present invention, wherein the compound is provided in a therapeutic composition comprising one or two additional compounds selected from: (a) A Receptor-interacting serine/threonine-protein kinase **1** (RIPK1) inhibitor, (b) An Inhibitor of κB (IκB) Kinase (IKK)/Nuclear Factor κB (NFκB) - signaling inhibitor, and (c) A B-cell lymphoma 2 (BCL-2) inhibitor, or wherein the compound is provided in a therapeutic composition comprising all of (a) to (c).

Yet another preferred embodiment of this invention relates to the compound for use according to this invention, wherein the RIPK1 inhibitor (a) is Nec-is, the IKK/NFκB - signaling inhibitor (b) is ML120B or LY2409881, and the BCL-2 inhibitor (c) is venetoclax, or any derivative, analogue, or salt of these compounds.

As will be further described herein, the subject or patient subjected to the treatments or uses of the invention in preferred embodiments is suffering from a proliferative disease, wherein the proliferative disease is preferably a solid cancer or a hematologic cancer, for example including leukemias, lymphomas, and myelomas.

In a further preferred embodiment, the proliferative disease to be treated and/or prevented according to this invention is preferably a cancer selected from B cell non-Hodgkin's lymphoma (B-NHL), T cell non-Hodgkin's lymphoma (T-NHL), acute myeloid leukemia (AML), acute lymphoblastic leukemia (ALL), such as B-cell ALL or T-cell ALL, for example precursor B-cell ALL, precursor T-cell ALL, mature B-cell ALL, or mature T-cell ALL, chronic lymphocytic leukemia (CLL), small lymphocytic lymphoma (SLL), Hodgkin's lymphoma, multiple myeloma, myelodysplastic syndrome (MDS), mantle-cell lymphoma (MCL), diffuse large B-cell lymphoma, activated B cell type diffuse large B cell lymphoma (ABC-DLBCL), cluster 1 diffuse large B cell lymphoma, and cluster 5 diffuse large B cell lymphoma; and preferably is B cell non-Hodgkin's lymphoma (B-NHL) and/or B-cell mature acute lymphoblastic leukemia (B-cell ALL). Particularly preferred is the treatment of indolent and aggressive B cell non-Hodgkin's lymphoma (B-NHL). Further preferred diseases or conditions to be treated and/or prevented are genetically classified in Chapuy et al., Nature Medicine, 2018 (19).

In an additionally preferred embodiment, the compound for use in the treatment and/or prevention of a proliferative disease in a subject, which is selected from: a) A Receptor-interacting serine/threonine-protein kinase 1 (RIPK1) inhibitor, (b) An Inhibitor of κB (IκB) Kinase (IKK)/Nuclear Factor κB (NFκB) - signaling inhibitor, and (c) A B-cell lymphoma 2 (BCL-2) inhibitor, wherein if the compound is (a), the subject will receive, receives or has received an additional treatment with (b) and (c), or if the compound is (b), the subject will receive, receives or has received an additional treatment with (a) and (c), or if the compound is (c), the subject will receive, receives or has received an additional treatment with (a) and (b), is used as a first line treatment in the treatment and/or prevention of a proliferative disease in a subject. In an additionally preferred embodiment, the compound, composition or combination for use according to this invention can be used prior additional treatment regimens. Any additional cancer treatments can be used according to this invention. As an example, the compound, composition or combination for use according to this invention can be used as a first line treatment regimen prior a subsequent treatment, such as a subsequent R-CHOP treatment regimen.

"R-CHOP" in the context of this invention shall pertain to a chemotherapy regimen used in the treatment of, for example, non-Hodgkin lymphoma. In general, R-CHOP comprises treatment with:
(R) Rituximab, a chimeric anti-CD20 monoclonal antibody,
(C) Cyclophosphamide, an alkylating agent which damages DNA by binding to it and causing the formation of cross-links,
(H) Hydroxydaunorubicin (also called doxorubicin or adriamycin), an intercalating agent which damages DNA by inserting itself between DNA bases,
(O) Oncovin (vincristine), which prevents cells from duplicating by binding to the protein tubulin, and
(P) Prednisone or prednisolone, which is a corticosteroid.

Importantly, any of the treatment regimens of this invention can be repeated any number of times. For example, the first treatment regimen comprising: a) A Receptor-interacting serine/threonine-protein kinase 1 (RIPK1) inhibitor, (b) an Inhibitor of κB (IκB) Kinase (IKK)/Nuclear Factor κB (NFκB) - signaling inhibitor, and (c) a B-cell lymphoma 2 (BCL-2) inhibitor, can be repeated once, twice, 3, 4, 5, 6, 7, 8, 9, 10, 11, 12, 13, 14, 15, 16, 17, 18, 19, 20, or any other number of times. Then, the second line of treatment, can also be repeated once, twice, 3, 4, 5, 6, 7, 8, 9, 10, 11, 12, 13, 14, 15, 16, 17, 18, 19, 20, or any other number of times. For example, if the second treatment regimen is R-CHOP, then the R-CHOP treatment can be repeated once, twice, 3, 4, 5, 6, 7, 8, 9, 10, 11, 12, 13, 14, 15, 16, 17, 18, 19, 20, or any other number of times. The second line treatment regimen can then be followed by a third line treatment regimen, a fourth line treatment regimen, a fifth line treatment regimen, a sixth line treatment regimen, a seventh line treatment regimen, an eight line treatment regimen, and so on.

Yet another preferred embodiment relates to the compound for use in the treatment and/or prevention of a proliferative disease in a subject, wherein the proliferative disease is a cancer which is refractory or resistant to treatment with at least one prior therapy.

In context of the present invention the term "subject" or "patient" preferably refers to a mammal, such as a mouse, rat, guinea pig, rabbit, cat, dog, monkey, or preferably a human, for example a human patient. The subject of the invention may be at danger of suffering from a proliferative disease. In a particularly preferred embodiment, the subject is a human patient suffering from a proliferative disease.

In another aspect, which can be combined with any of the other aspects and any of the particularly preferred embodiments, the present invention provides a pharmaceutical composition for use in the treatment and/or prevention of a proliferative disease in a subject, wherein the pharmaceutical composition comprises a RIPK1 inhibitor, an IKK/NFκB - signaling inhibitor, and a BCL-2 inhibitor as described before, together with a pharmaceutically acceptable carrier and/or excipient.

The pharmaceutical composition of the invention is formulated to be compatible with its intended route of administration. Examples of routes of administration include parenteral, e.g., intrathecal, intra-arterial, intravenous, intradermal, subcutaneous, oral, transdermal (topical), intracerebroventricular, intraparenchymal, intratumoral and transmucosal administration. Generally a pharmaceutical composition of the invention shall comprise one or more compounds for inhibiting RIPK1-, IKK/NFκB - signaling, and BCL-2, and at least one pharmaceutically acceptable carrier and/or excipient. The pharmaceutical compositions as described herein are particularly useful for use in the herein described methods for treating proliferative diseases.

The term "intrathecal," as used herein, means introduced into or occurring in the space under the arachnoid membrane which covers the brain and spinal cord. The term "intracerebroventricular" refers to administration of a composition into the ventricular system of the brain, e.g., via injection, infusion, or implantation (for example, into a ventricle of the brain). As used herein, the term "intraparenchymal" can refer to an administration directly to brain tissue. In other instances, intraparenchymal administration may be directed to any brain region where delivery of one or more compounds of the invention is effective to mitigate or prevent one or more of diseases and/or disorders as described herein.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; anti-bacterial agents such as benzyl alcohol or methyl parabens; antioxidants, such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a sulfotransferase inhibitor) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying, which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g, a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions are formulated into ointments, salves, gels, or creams as generally known in the art.

In certain embodiments, the pharmaceutical composition is formulated for sustained or controlled release of the active ingredient. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from e.g. Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of ad-ministration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. The pharmaceutical compositions can be included in a container, pack, or dispenser together with instructions for administration.

In a particularly preferred embodiment, the pharmaceutical composition of the invention comprises a RIPK1 inhibitor selected from the group consisting of necrostatin-1 stable (5-((7-chloro-1H-indol-3-yl)methyl)-3-methyl-2,4-imidazolidinedione), necrostatin-1(5-(1H-indo1-3-ylmethyl)-3-methyl-2-thioxo-4-imidazolidinone), necrostatin-2(5-[(7-chloro-1H-indol-3-yl)methyl]-3-methyl-2,4-imidazolidinedione), necrostatin-3(3-phenyl-3,3a,4,5-tetrahydro-2H-benz[g]indazole, 3-phenyl-2,3,3a,4-tetrahydro[1]benzopyrano[4,3-c]pyrazole, 3-phenyl-2,3,3a,4-tetrahydro[1]benzothiopyrano[4,3-c]pyrazole, 5,5-dioxo-3-phenyl-2,3,3a,4-tetrahydro[1]benzothiopyrano[4,3-c]pyrazole]), necrostatin-4((S)-N-(1-[2-chloro-6-fluorophenyl]ethyl)-5-cyano-1-methyl-1H-pyrrole-2-carboxamide), necrostatin-5(2-[[3,4,5,6,7,8-hexahydro-3-(4-methoxyphenyl)-4-oxo[1]benzothieno[2,3-d]pyrimidin-2-yl]thio]-acetonitrile), necrostatin-7(5-((3-(4-fluorophenyl)-1H-pyrazol-4-yl)methylene)-2-imino-3-(thiazol-2-yl)thiazolidin-4-one), necrostatin-21, (S)-5-benzyl-N-(7,9difluoro-2-oxo-2,3,4,5-tetrahydro-1H-benzo[b]azepin-3-yl)1H-1,2,4-triazole-3-carboxamide (GSK3145095), N-benzyl-N-hydroxy-2,2-dimethylbutanamide (RIPA-56), DNL747 (SAR 443060), (S)-*N*-(5-methyl-4-oxo-2,3,4,5-tetrahydrobenzo[*b*][1,4]oxazepin-3-yl)-4,5,6,7-tetrahydro-1*H*-indazole-3-carboxamide, (S)-*N*-(5-methyl-4-oxo-2,3,4,5-tetrahydrobenzo[*b*][1,4]oxazepin-3-yl)-4*H*-1,2,4-triazole-3-carboxamide, (*R*)-5-methyl-*N*-((S)-5-methyl-4-oxo-2,3,4,5-tetrahydrobenzo[*b*][1,4]oxazepin-3-yl)-4,5,6,7-tetrahydro-1H-indazole-3-carboxamide, (*R*)-5-methyl-*N*-((*S*)-5-methyl-4-oxo-2,3,4,5-tetrahydrobenzo[*b*][1,4]oxazepin-3-yl)-4,5,6,7-tetrahydro-1H-indazole-3-carboxamide, (S)-6,6-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (S)-N-(5-methyl-4-oxo-2,3,4,5-tetrahydrobenzo[b][1,4]oxazepin-3-yl)-1',4',5',7'-tetrahydrospiro[cyclopropane-1,6'-indazole]-3'-carboxamide, N-((S)-5-methyl-4-oxo-2,3,4,5-tetrahydrobenzo[b][1,4]oxazepin-3-yl)-1,4,4a,5,5a,6-hexahydrocyclopropa[f]indazole-3-carboxamide, N-((S)-5-methyl-4-oxo-2,3,4,5-tetrahydrobenzo[b][1,4]oxazepin-3-yl)-3,4,5,5a,6,6a-hexahydrocyclopropa[e]indazole-1-carboxamide, (S)-5,5-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (R)-5-(tert-butyl)-N-((S)-5-methyl-4-oxo-2,3,4,5-tetrahydrobenzo[b][1,4]oxazepin-3-yl)-4,5,6,7-tetrahydro-1H-indazole-3-carboxamide, N-((S)-5-methyl-4-oxo-2,3,4,5-tetrahydrobenzo[b][1,4]oxazepin-3-yl)-5-phenyl-4,5,6,7-tetrahydro-1H-indazole-3-carboxamide, N-((S)-5-methyl-4-oxo-2,3,4,5-tetrahydrobenzo[b][1,4]oxazepin-3-yl)-5-(1H-pyrazol-1-yl)-4,5,6,7-tetrahydro-1H-indazole-3-carboxamide, (R)-1-methyl-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (S)-N-(5-methyl-4-oxo-2,3,4,5-tetrahydrobenzo[b][1,4]oxazepin-3-yl)-5-(trifluoromethyl)-1H-indazole-3-carboxamide, (S)-N-(5-methyl-4-oxo-2,3,4,5-tetrahydrobenzo[b][1,4]oxazepin-3-yl)-5-(trifluoromethyl)-1H-pyrazolo[3,4-b]pyridine-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-[1,2,3]triazolo [1,5-a]pyridine-3-carboxamide, (S)-N-(5-methyl-4-oxo-2,3,4,5-tetrahydrobenzo[b][1,4]oxazepin-3-yl)-7-(trifluoromethyl)imidazo[1,5-a]pyridine-1-carboxamide, (S)-N-(5-methyl-4-oxo-2,3,4,5-tetrahydrobenzo[b][1,4]oxazepin-3-yl)-6-(trifluoromethyl)-[1,2,4]triazolo[4,3-b]pyridazine-3-carboxamide, (S)-5-isopropyl-N-(5-methyl-4-oxo-2,3,4,5-tetrahydrobenzo[b][1,4]oxazepin-3-yl)-1H-pyrazolo[3,4-b]pyridine-3-carboxamide, (S)-N-(5-methyl-4-oxo-2,3,4,5-tetrahydrobenzo[b][1,4]oxazepin-3-yl)-5-(perfluoroethyl)-1H-pyrazolo[3,4-b]pyridine-3-carboxamide, N-((S)-5-methyl-4-oxo-2,3,4,5-tetrahydrobenzo[b][1,4]oxazepin-3-yl)-5-(perfluoroethyl)-4,5,6,7-tetrahydro-[1,2,3]triazolo[1,5-a]pyridine-3-carboxamide, (S)-N-(5-methyl-4-oxo-2,3,4,5-tetrahydrobenzo[b][1,4]oxazepin-3-yl)-6-(perfluoroethyl)-[1,2,4]triazolo[4,3-b]pyridazine-3-carboxamide, (S)-5-benzyl-N-(5-methyl-4-oxo-2,3,4,5-tetrahydrobenzo[b][1,4]oxazepin-3-yl)isoxazole-3-carboxamide (GSK481), 1-1(5S)-4,5-Dihydro-5-phenyl-1H-pyrazol-1-yl]-2,2-dimethyl-1-propanone, 2,2-Dimethyl-1-(5(S)-phenyl-4,5-dihydro-pyrazol-1-yl)-propan-1-one (GSK963), (S)-5-benzyl-N-(5-methyl-4-oxo-2,3,4,5-tetrahydrobenzo[b][1,4]oxazepin-3-yl)-1H-1,2,4-triazole-3-carboxamide (GSK2982772), 5-(indol-3-ylmethyl)-3-methyl-2-thio-hydantoin), and their derivatives or analogues. In a further preferred embodiment, the RIPK1 inhibitor is necrostatin-1 stable (Nec-1s).

Yet another particularly preferred embodiment relates to the pharmaceutical composition of this invention, wherein said pharmaceutical composition comprises an IKK/NFκB signaling inhibitor, which is an IKK inhibitor, preferably an IKK2/IKKβ inhibitor. In a further preferred embodiment, said IKK2/IKKβ inhibitor is selected from the group consisting of N-(6-chloro-7-methoxy-9H-beta-carbolin-8-yl)-2-methylnicotinamide (ML120B), LY2409881, (3S)-N-(6-Chloro-9H-beta-carbolin-8-yl)-4-(2-((2R,6S)-2,6-dimethylmorpholin-4-yl)-2-oxoethyl)-6,6-dimethylmorpholine-3-carboxamide (MLN-0415), 2-(pyrimidin-4-yl)-1H-indole (SAR-113945), 1-(5-Methoxy-2-thiophen-2-yl-quinazolin-4-ylamino)-3-methyl-pyrrole-2,5-dione (CDC-839), 1H-imidazo[4,5-b]pyridin-5-amine, 7-[5-[(cyclohexylmethylamino)-methyl]-1H-indol-2-yl]-2-methyl, sulfate trihydrate (E-6070), TPCA-1, Bardoxolone Methyl, Resveratrol, MRT67307, AmLexanox, BMS-345541, IKK 16, BI605906, ACHP Hydrochloride, PS-1145, Apigenin, SC-514, IKKε-IN-1, IMD-0354, AZD 3264, Icariin (Ieariline), Bay 65-1942 Hcl, Bay 65-1942, an Anilino-Pyrimidine, IKK inhibitor III, SC-514*Amino-imidazolecarboxamide, an Ureudo-thiophenecarboxamide, a Diarylpybidine, a Pyridooxazinone, an Indolecarboxamide, a Benzoimidazole carboxamide, a Pyrazolo[4,3-c]quinoline, an Imidazolylquinoline-carbxaldehyde semicarbazide, a Pyridyl Cyanoguanidine, IkB Kinase Inhibitor Peptide, IKK-2 Inhibitor IV ([5-(p-Fluorophenyl)-2-ureido]thiophene-3-carboxamide), IKK Inhibitor II, Wedelolactone, IKK Inhibitor VII, IKK-2 Inhibitor V (N-(3,5-Bis-trifluoromethylphenyl)-5-chloro-2-hydroxybenzamide), IKK-2 Inhibitor VI (5- Phenyl-2-ureido)thiophene-3-carboxamide), IKK-2 Inhibitor VIII ACHP (2-Amino-6-(2-(cyclopropylmethoxy)-6-hydroxyphenyl)-4-(4-piperidinyl)-3-pyridinecarbonitrile), and their derivatives or analogues. In yet another preferred embodiment, the IKK inhibitor is ML120B or LY2409881.

A further preferred embodiment relates to the pharmaceutical composition of this invention, wherein said pharmaceutical composition comprises a BCL-2 inhibitor, which is selected from the group consisting of 4-[4-[[2-(4-chlorophenyl)-5,5-dimethyl-1-cyclohexen-1-yl]methyl]-1-piperazinyl]-N-[[4-[[(1R)-3-(4-morpholinyl)-1-[(phenylthio)methyl]propyl]amino]-3-[(trifluoromethyl)sulfonyl]phenyl] sulfonyl]benzamide (navitoclax or ABT-263), tetrocarcin A, antimycin, gossypol (such as (-)-gossypol acetic acid), obatoclax (GX15-070), ethyl 2-amino-6-bromo-4-(1-cyano-2-ethoxy-2-oxoethyl)-4H-chromene-3-carboxylate (HA 14-1), oblimersen, a Bak BH3 peptide, 4-[4-[(4'-chloro[1,1'-biphenyl]-2-yl)methyl]-1-piperazinyl]-N-[[4-[[(1R)-3-(dimethylamino)-1-[(phenylthio)methyl]propyl]amino]-3-nitrophenyl]sulfonyl]-benzamide (ABT-737), 2-((1H-pyrrolo[2,3-b]pyridin-5-yl)oxy)-4-(44(4'-chloro-5,5-dimethyl-3,4,5,6-tetrahydro-[1,1'-biphenyl]-2-Amethyl)piperazin-1-yl)-N-((3-nitro-4-(((tetrahydro-2H-pyran-4-Amethyl)amino)phenyl)sulfonyl)benzamide (venetoclax), S55746 (BCL201), and their derivatives or analogues. In a particularly preferred embodiment, the BCL-2 inhibitor is venetoclax.

In a further preferred embodiment, the pharmaceutical composition for use according to the present invention comprises a RIPK1 inhibitor, an IKK/NFκB - signaling inhibitor, and a BCL-2 inhibitor, wherein the RIPK1 inhibitor is Nec-is, the IKK/NFκB - signaling inhibitor is ML120B or LY2409881, and the BCL-2 inhibitor is venetoclax, or any derivative, analogue, or salt of these compounds.

In another preferred embodiment, the pharmaceutical composition is for use in the treatment and/or prevention of a proliferative disease in a subject, wherein the proliferative disease is preferably a solid cancer or a hematologic cancer, for example including leukemias, lymphomas, and myelomas.

In a further preferred embodiment, the proliferative disease to be treated and/or prevented using the pharmaceutical composition for use according to this invention is preferably a cancer selected from B cell non-Hodgkin's lymphoma (B-NHL), T cell non-Hodgkin's lymphoma (T-NHL), acute myeloid leukemia (AML), acute lymphoblastic leukemia (ALL), such as B-cell ALL or T-cell ALL, for example precursor B-cell ALL, precursor T-cell ALL, mature B-cell ALL, or mature T-cell ALL, chronic lymphocytic leukemia (CLL), small lymphocytic lymphoma (SLL), Hodgkin's lymphoma, multiple myeloma, myelodysplastic syndrome (MDS), mantle-cell lymphoma (MCL), diffuse large B-cell lymphoma, activated B cell type diffuse large B cell lymphoma (ABC-DLBCL), cluster 1 diffuse large B cell lymphoma, and cluster 5 diffuse large B cell lymphoma; and preferably is B cell non-Hodgkin's lymphoma (B-NHL) and/or B-cell mature acute lymphoblastic leukemia (B-cell ALL).

In a further aspect of this invention, which can be combined with any one of the other aspects and/or embodiments of this invention, a combination (of compounds) for use in the treatment and/or prevention of a proliferative disease is provided, wherein the combination comprises a RIPK1 inhibitor, an IKK/NFκB signaling inhibitor, and a BCL-2 inhibitor as defined herein. Preferably the treatment and/or prevention comprises the administration of a therapeutically effective amount of a RIPK1 inhibitor, an IKK/NFκB signaling inhibitor, and a BCL-2 inhibitor to a subject suffering from the proliferative disease.

In a particularly preferred embodiment, the combination (of compounds) for use in the treatment and/or prevention of a proliferative disease of the present invention comprises a RIPK1 inhibitor selected from the group consisting of necrostatin-1 stable (5-((7-chloro-1H-indol-3-yl)methyl)-3-methyl-2,4-imidazolidinedione), necrostatin-1(5-(1H-indo1-3-ylmethyl)-3-methyl-2-thioxo-4-imidazolidinone), necrostatin-2(5-[(7-chloro-1H-indol-3-yl)methyl]-3-methyl-2,4-imidazolidinedione), necrostatin-3(3-phenyl-3,3a,4,5-tetrahydro-2H-benz[g]indazole, 3-phenyl-2,3,3a,4-tetrahydro[1]benzopyrano[4,3-c]pyrazole, 3-phenyl-2,3,3a,4-tetrahydro[1]benzothiopyrano[4,3-c]pyrazole, 5,5-dioxo-3-phenyl-2,3,3a,4-tetrahydro[1]benzothiopyrano[4,3-c]pyrazole]), necrostatin-4((S)-N-(1-[2-chloro-6-fluorophenyl]ethyl)-5-cyano-1-methyl-1H-pyrrole-2-carboxamide), necrostatin-5(2-[[3,4,5,6,7,8-hexahydro-3-(4-methoxyphenyl)-4-oxo[1]benzothieno[2,3-d]pyrimidin-2-yl]thio]-acetonitrile), necrostatin-7(5-((3-(4-fluorophenyl)-1H-pyrazol-4-yl)methylene)-2-imino-3-(thiazol-2-yl)thiazolidin-4-one), necrostatin-21, (S)-5-benzyl-N-(7,9difluoro-2-oxo-2,3,4,5-tetrahydro-1H-benzo[b]azepin-3-yl)1H-1,2,4-triazole-3-carboxamide (GSK3145095), N-benzyl-N-hydroxy-2,2-dimethylbutanamide (RIPA-56), DNL747 (SAR 443060), (*S*)-*N*-(5-methyl-4-oxo-2,3,4,5-etrahydrobenzo[*b*][1,4]oxazepin-3-yl)-4,56,7-tetrahydro-1*H*-indazole-3-carboxamide, (S)-*N*-(5-methyl-4-oxo-2,3,4,5-tetrahydrobenzo[*b*][1,4]oxazepin-3-yl)-4*H*-1,2,4-triazole-3-carboxamide, (*R*)-5-methyl-*N*-((*S*)-5-methyl-4-oxo-3,4,5-tetrahydrobenzo[*b*][1,4]oxazepin-3-yl)-4,5,6,7-tetrahydro-1H-indazole-3-carboxamide, (*R*)-5-methyl-*N*-((*S*)-5-methyl-4-oxo-2,3,4,5-tetrahydrobenzo[*b*][1,4]oxazepin-3-yl)-4,5,6,7-tetrahydro-1H-indazole-3-carboxamide, (S)-6,6-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (S)-N-(5-methyl-4-oxo-2,3,4,5-tetrahydrobenzo[b][1,4]oxazepin-3-yl)-1',4',5',7'-tetrahydrospiro[cyclopropane-1,6'-indazole]-3'-carboxamide, N-((S)-5-methyl-4-oxo-2,3,4,5-tetrahydrobenzo[b][1,4]oxazepin-3-yl)-1,4,4a,5,5a,6-hexahydrocyclopropa[f]indazole-3-carboxamide, N-((S)-5-methyl-4-oxo-2,3,4,5-tetrahydrobenzo[b][1,4]oxazepin-3-yl)-3,4,5,5a,6,6a-hexahydrocyclopropa[e]indazole-1-carboxamide, (S)-5,5-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (R)-5-(tert-butyl)-N-((S)-5-methyl-4-oxo-2,3,4,5-tetrahydrobenzo[b][1,4]oxazepin-3-yl)-4,5,6,7-tetrahydro-1H-indazole-3-carboxamide, N-((S)-5-methyl-4-oxo-2,3,4,5-tetrahydrobenzo[b][1,4]oxazepin-3-yl)-5-phenyl-4,5,6,7-tetrahydro-1H-indazole-3-carboxamide, N-((S)-5-methyl-4-oxo-2,3,4,5-tetrahydrobenzo[b][1,4]oxazepin-3-yl)-5-(1H-pyrazol-1-yl)-4,5,6,7-tetrahydro-1H-indazole-3-carboxamide, (R)-1-methyl-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (S)-N-(5-methyl-4-oxo-2,3,4,5-tetrahydrobenzo[b][1,4]oxazepin-3-yl)-5-(trifluoromethyl)-1H-indazole-3-carboxamide, (S)-N-(5-methyl-4-oxo-2,3,4,5-tetrahydrobenzo[b][1,4]oxazepin-3-yl)-5-(trifluoromethyl)-1H-pyrazolo[3,4-b]pyridine-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-[1,2,3]triazolo [1,5-a]pyridine-3-carboxamide, (S)-N-(5-methyl-4-oxo-2,3,4,5-tetrahydrobenzo[b][1,4]oxazepin-3-yl)-7-(trifluoromethyl)imidazo[1,5-a]pyridine-1-carboxamide, (S)-N-(5-methyl-4-oxo-2,3,4,5-tetrahydrobenzo[b][1,4]oxazepin-3-yl)-6-(trifluoromethyl)-[1,2,4]triazolo[4,3-b]pyridazine-3-carboxamide, (S)-5-isopropyl-N-(5-methyl-4-oxo-2,3,4,5-tetrahydrobenzo[b][1,4]oxazepin-3-yl)-1H-pyrazolo[3,4-b]pyridine-3-carboxamide, (S)-N-(5-methyl-4-oxo-2,3,4,5-tetrahydrobenzo[b][1,4]oxazepin-3-yl)-5-(perfluoroethyl)-1H-pyrazolo[3,4-b]pyridine-3-carboxamide, N-((S)-5-methyl-4-oxo-2,3,4,5-tetrahydrobenzo[b][1,4]oxazepin-3-yl)-5-(perfluoroethyl)-4,5,6,7-tetrahydro-[1,2,3]triazolo[1,5-a]pyridine-3-carboxamide, (S)-N-(5-methyl-4-oxo-2,3,4,5-tetrahydrobenzo[b][1,4]oxazepin-3-yl)-6-(perfluoroethyl)-[1,2,4]triazolo[4,3-b]pyridazine-3-carboxamide, (S)-5-benzyl-N-(5-methyl-4-oxo-2,3,4,5-tetrahydrobenzo[b][1,4]oxazepin-3-yl)isoxazole-3-carboxamide (GSK481), 1-[(5S)-4,5-Dihydro-5-phenyl-1H-pyrazol-1-yl]-2,2-dimethyl-1-propanone, 2,2-Dimethyl-1-(5(S)-phenyl-4,5-dihydro-pyrazol-1-yl)-propan-1-one (GSK963), (S)-5-benzyl-N-(5-methyl-4-oxo-2,3,4,5-tetrahydrobenzo[b][1,4]oxazepin-3-yl)-1H-1,2,4-triazole-3-carboxamide (GSK2982772), 5-(indol-3-ylmethyl)-3-methyl-2-thio-hydantoin), and their derivatives or analogues. In a further preferred embodiment, the RIPK1 inhibitor is necrostatin-1 stable (Nec-1s).

Yet another particularly preferred embodiment relates to the combination (of compounds) for use in the treatment and/or prevention of a proliferative disease, wherein said combination comprises an IKK/NFκB signaling inhibitor, which is an IKK inhibitor, preferably an IKK2/IKKβ inhibitor. In a further preferred embodiment, said IKK2/IKKβ inhibitor is selected from the group consisting of N-(6-chloro-7-methoxy-9H-beta-carbolin-8-yl)-2-methylnicotinamide (ML120B), LY2409881, (3S)-N-(6-Chloro-9H-beta-carbolin-8-yl)-4-(2-((2R,6S)-2,6-dimethylmorpholin-4-yl)-2-oxoethyl)-6,6-dimethylmorpholine-3-carboxamide (MLN-0415), 2-(pyrimidin-4-yl)-1H-indole (SAR-113945), 1-(5-Methoxy-2-thiophen-2-yl-quinazolin-4-ylamino)-3-methyl-pyrrole-2,5-dione (CDC-839), 1H-imidazo[4,5-b]pyridin-5-amine, 7-[5-[(cyclohexylmethylamino)-methyl]-1H-indol-2-yl]-2-methyl, sulfate trihydrate (E-6070), TPCA-1, Bardoxolone Methyl, Resveratrol, MRT67307, AmLexanox, BMS-345541, IKK 16, BI605906, ACHP Hydrochloride, PS-1145, Apigenin, SC-514, IKKε-IN-1, IMD-0354, AZD 3264, Icariin (Ieariline), Bay 65-1942 Hcl, Bay 65-1942, an Anilino-Pyrimidine, IKK inhibitor III, SC-514*Amino-imidazolecarboxamide, an Ureudo-thiophenecarboxamide, a Diarylpybidine, a Pyridooxazinone, an Indolecarboxamide, a Benzoimidazole carboxamide, a Pyrazolo[4,3-c]quinoline, an Imidazolylquinoline-carbxaldehyde semicarbazide, a Pyridyl Cyanoguanidine, IkB Kinase Inhibitor Peptide, IKK-2 Inhibitor IV ([5-(p-Fluorophenyl)-2-ureido]thiophene-3-carboxamide), IKK Inhibitor II, Wedelolactone, IKK Inhibitor VII, IKK-2 Inhibitor V (N-(3,5-Bis-trifluoromethylphenyl)-5-chloro-2-hydroxybenzamide), IKK-2 Inhibitor VI (5-Phenyl-2-ureido)thiophene-3-carboxamide), IKK-2 Inhibitor VIII ACHP (2-Amino-6-(2-(cyclopropylmethoxy)-6-hydroxyphenyl)-4-(4-piperidinyl)-3-pyridinecarbonitrile), and their derivatives or analogues. In yet another preferred embodiment, the IKK inhibitor is ML120B or LY2409881.

A further preferred embodiment relates to the combination (of compounds) for use in the treatment and/or prevention of a proliferative disease, wherein said combination comprises a BCL-2 inhibitor, which is selected from the group consisting of 4-[4-[[2-(4-chlorophenyl)-5,5-dimethyl-1-cyclohexen-1-yl]methyl]-1-piperazinyl]-N-[[4-[[(1R)-3-(4-morpholinyl)-1-[(phenylthio)methyl]propyl]amino]-3-[(trifluoromethyl)sulfonyl]phenyl] sulfonyl]benzamide (navitoclax or ABT-263), tetrocarcin A, antimycin, gossypol (such as (-)-gossypol acetic acid), obatoclax (GX15-070), ethyl 2-amino-6-bromo-4-(1-cyano-2-ethoxy-2-oxoethyl)-4H-chromene-3-carboxylate (HA 14-1), oblimersen, a Bak BH₃ peptide, 4-[4-[(4'-chloro[1,1'-biphenyl]-2-yl)methyl]-1-piperazinyl]-N-[[4-[[(1R)-3-(dimethylamino)-1-[(phenylthio)methyl]propyl]amino]-3-nitrophenyl]sulfonyl]-benzamide (ABT-737), 2-((1H-pyrrolo[2,3-b]pyridin-5-yl)oxy)-4-(44(4'-chloro-5,5-dimethyl-3,4,5,6-tetrahydro-[1,1'-biphenyl]-2-Amethyl)piperazin-1-yl)-N-((3-nitro-4-(((tetrahydro-2H-pyran-4-Amethyl)amino)phenyl)sulfonyl)benzamide (venetoclax), S55746 (BCL201), and their derivatives or analogues. In a particularly preferred embodiment, the BCL-2 inhibitor is venetoclax.

In a particularly preferred embodiment, the combination for use according to the present invention comprises a RIPK1 inhibitor, an IKK/NFκB - signaling inhibitor, and a BCL-2 inhibitor, wherein the RIPK1 inhibitor is Nec-is, the IKK/NFκB - signaling inhibitor is ML120B or LY2409881, and the BCL-2 inhibitor is venetoclax, or any derivative, analogue, or salt of these compounds.

The combination for use according to the present invention, is particularly for the use of treating and/or preventing a proliferative disease, wherein the proliferative disease is preferably a solid cancer or a hematologic cancer, for example including leukemias, lymphomas, and myelomas.

Further preferred is a combination according to this invention for the use of treating and/or preventing a proliferative disease, wherein the proliferative disease is a cancer selected from B cell non-Hodgkin's lymphoma (B-NHL), T cell non-Hodgkin's lymphoma (T-NHL), acute myeloid leukemia (AML), acute lymphoblastic leukemia (ALL), such as B-cell ALL or T-cell ALL, for example precursor B-cell ALL, precursor T-cell ALL, mature B-cell ALL, or mature T-cell ALL, chronic lymphocytic leukemia (CLL), small lymphocytic lymphoma (SLL), Hodgkin's lymphoma, multiple myeloma, myelodysplastic syndrome (MDS), mantle-cell lymphoma (MCL), diffuse large B-cell lymphoma, activated B cell type diffuse large B cell lymphoma (ABC-DLBCL), cluster 1 diffuse large B cell lymphoma, and cluster 5 diffuse large B cell lymphoma; and preferably is B cell non-Hodgkin's lymphoma (B-NHL) and/or B-cell mature acute lymphoblastic leukemia (B-cell ALL).

A further aspect of this invention, which can be combined with any one of the other aspects and/or embodiments of this invention, pertains to the use of
**(a)** A Receptor-interacting serine/threonine-protein kinase 1 (RIPK1) inhibitor;
(b)An Inhibitor of κB (IκB) Kinase (IKK)/Nuclear Factor κB (NFκB) - signaling inhibitor; and
(c) A B-cell lymphoma 2 (BCL-2) inhibitor,
for the manufacture of a medicament for the treatment and/or prevention of a proliferative disease.

In a particularly preferred embodiment of this invention a use as described above is preferred, wherein the RIPK1 inhibitor is selected from the group consisting of necrostatin-1 stable (5-((7-chloro-1H-indol-3-yl)methyl)-3-methyl-2,4-imidazolidinedione), necrostatin-1(5-(1H-indo1-3-ylmethyl)-3-methyl-2-thioxo-4-imidazolidinone), necrostatin-2(5-[(7-chloro-1H-indol-3-yl)methyl]-3-methyl-2,4-imidazolidinedione), necrostatin-3 (3-phenyl-3,3a,4,5-tetrahydro-2H-benz[g]indazole, 3-phenyl-2,3,3a,4-tetrahydro[1]benzopyrano[4,3-c]pyrazole, 3-phenyl-2,3,3a,4-tetrahydro[1]benzothiopyrano[4,3-c]pyrazole, 5,5-dioxo-3-phenyl-2,3,3a,4-tetrahydro[1]benzothiopyrano[4,3-c]pyrazole]), necrostatin-4((S)-N-(1-[2-chloro-6-fluorophenyl]ethyl)-5-cyano-1-methyl-1H-pyrrole-2-carboxamide), necrostatin-5(2-[[3,4,5,6,7,8-hexahydro-3-(4-methoxyphenyl)-4-oxo[1]benzothieno[2,3-d]pyrimidin-2-yl]thio]-acetonitrile), necrostatin-7(5-((3-(4-fluorophenyl)-1H-pyrazol-4-yl)methylene)-2-imino-3-(thiazol-2-yl)thiazolidin-4-one), necrostatin-21, (S)-5-benzyl-N-(7,9difluoro-2-oxo-2,3,4,5-tetrahydro-1H-benzo[b]azepin-3-yl)1H-1,2,4-triazole-3-carboxamide (GSK3145095), N-benzyl-N-hydroxy-2,2-dimethylbutanamide (RIPA-56), DNL747 (SAR 443060), (S)-N-(5-methyl-4-oxo-2,3,4,5-tetrahydrobenzo[*b*][1,4]oxazepin-3-yl)-4,5,6,7-tetrahydro-1*H*-indazole-3-carboxamide, (*S*)-*N*-(5-methyl-4-oxo-2,3,4,5-tetrahydrobenzo[*b*][1,4]oxazepin-3-yl)-4*H*-1,2,4-triazole-3-carboxamide, (*R*)-5-methyl-*N*-((*S*)-5-methyl-4-oxo-2,3,4,5-tetrahydrobenzo[*b*][1,4]oxazepin-3-yl)-4,5,6,7-tetrahydro-1H-indazole-3-carboxamide, (*R*)-5-methyl-*N*-((*S*)-5-methyl-4-oxo-2,3,4,5-tetrahydrobenzo[*b*][1,4]oxazepin-3-yl)-4,5,6,7-tetrahydro-1H-indazole-3-carboxamide, (S)-6,6-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (S)-N-(5-methyl-4-oxo-2,3,4,5-tetrahydrobenzo[b][1,4]oxazepin-3-yl)-1',4',5',7'-tetrahydrospiro[cyclopropane-1,6'-indazole]-3'-carboxamide, N-((S)-5-methyl-4-oxo-2,3,4,5-tetrahydrobenzo[b][1,4]oxazepin-3-yl)-1,4,4a,5,5a,6-hexahydrocyclopropa[f]indazole-3-carboxamide, N-((S)-5-methyl-4-oxo-2,3,4,5-tetrahydrobenzo[b][1,4]oxazepin-3-yl)-3,4,5,5a,6,6a-hexahydrocyclopropa[e]indazole-1-carboxamide, (S)-5,5-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (R)-5-(tert-butyl)-N-((S)-5-methyl-4-oxo-2,3,4,5-tetrahydrobenzo[b][1,4]oxazepin-3-yl)-4,5,6,7-tetrahydro-1H-indazole-3-carboxamide, N-((S)-5-methyl-4-oxo-2,3,4,5-tetrahydrobenzo[b][1,4]oxazepin-3-yl)-5-phenyl-4,5,6,7-tetrahydro-1H-indazole-3-carboxamide, N-((S)-5-methyl-4-oxo-2,3,4,5-tetrahydrobenzo[b][1,4]oxazepin-3-yl)-5-(1H-pyrazol-1-yl)-4,5,6,7-tetrahydro-1H-indazole-3-carboxamide, (R)-i-methyl-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (S)-N-(5-methyl-4-oxo-2,3,4,5-tetrahydrobenzo[b][1,4]oxazepin-3-yl)-5-(trifluoromethyl)-1H-indazole-3-carboxamide, (S)-N-(5-methyl-4-oxo-2,3,4,5-tetrahydrobenzo[b][1,4]oxazepin-3-yl)-5-(trifluoromethyl)-1H-pyrazolo[3,4-b]pyridine-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-[1,2,3]triazolo [1,5-a]pyridine-3-carboxamide, (S)-N-(5-methyl-4-oxo-2,3,4,5-tetrahydrobenzo[b][1,4]oxazepin-3-yl)-7-(trifluoromethyl)imidazo[1,5-a]pyridine-1-carboxamide, (S)-N-(5-methyl-4-oxo-2,3,4,5-tetrahydrobenzo[b][1,4]oxazepin-3-yl)-6-(trifluoromethyl)-[1,2,4]triazolo[4,3-b]pyridazine-3-carboxamide, (S)-5-isopropyl-N-(5-methyl-4-oxo-2,3,4,5-tetrahydrobenzo[b][1,4]oxazepin-3-yl)-1H-pyrazolo[3,4-b]pyridine-3-carboxamide, (S)-N-(5-methyl-4-oxo-2,3,4,5-tetrahydrobenzo[b][1,4]oxazepin-3-yl)-5-(perfluoroethyl)-1H-pyrazolo[3,4-b]pyridine-3-carboxamide, N-((S)-5-methyl-4-oxo-2,3,4,5-tetrahydrobenzo[b][1,4]oxazepin-3-yl)-5-(perfluoroethyl)-4,5,6,7-tetrahydro-[1,2,3]triazolo[1,5-a]pyridine-3-carboxamide, (S)-N-(5-methyl-4-oxo-2,3,4,5-tetrahydrobenzo[b][1,4]oxazepin-3-yl)-6-(perfluoroethyl)-[1,2,4]triazolo[4,3-b]pyridazine-3-carboxamide, (S)-5-benzyl-N-(5-methyl-4-oxo-2,3,4,5-tetrahydrobenzo[b][1,4]oxazepin-3-yl)isoxazole-3-carboxamide (GSK481), 1-[(5S)-4,5-Dihydro-5-phenyl-1H-pyrazol-1-yl]-2,2-dimethyl-1-propanone, 2,2-Dimethyl-1-(5(S)-phenyl-4,5-dihydro-pyrazol-1-yl)-propan-1-one (GSK963), (S)-5-benzyl-N-(5-methyl-4-oxo-2,3,4,5-tetrahydrobenzo[b][1,4]oxazepin-3-yl)-1H-1,2,4-triazole-3-carboxamide (GSK2982772), 5-(indol-3-ylmethyl)-3-methyl-2-thio-hydantoin), and their derivatives or analogues. In a further preferred embodiment, the RIPK1 inhibitor is necrostatin-1 stable (Nec-1s).

Yet another particularly preferred embodiment relates to the use according to this invention, wherein the IKK/NFκB signaling inhibitor is an IKK inhibitor, preferably an IKK2/IKKβ inhibitor. In a particularly preferred embodiment, said IKK2/IKKβ inhibitor is selected from the group consisting of N-(6-chloro-7-methoxy-9H-beta-carbolin-8-yl)-2-methylnicotinamide (ML120B), LY2409881, (3S)-N-(6-Chloro-9H-beta-carbolin-8-yl)-4-(2-((2R,6S)-2,6-dimethylmorpholin-4-yl)-2-oxoethyl)-6,6-dimethylmorpholine-3-carboxamide (MLN-0415), 2-(pyrimidin-4-yl)-1H-indole (SAR-113945), 1-(5-Methoxy-2-thiophen-2-yl-quinazolin-4-ylamino)-3-methyl-pyrrole-2,5-dione (CDC-839), 1H-imidazo[4,5-b]pyridin-5-amine, 7-[5-[(cyclohexylmethylamino)-methyl]-1H-indol-2-yl]-2-methyl, sulfate trihydrate (E-6070), TPCA-1, Bardoxolone Methyl, Resveratrol, MRT67307, AmLexanox, BMS-345541, IKK 16, BI605906, ACHP Hydrochloride, PS-1145, Apigenin, SC-514, IKKε-IN-1, IMD-0354, AZD 3264, Icariin (Ieariline), Bay 65-1942 Hcl, Bay 65-1942, an Anilino-Pyrimidine, IKK inhibitor III, SC-514*Amino-imidazolecarboxamide, an Ureudo-thiophenecarboxamide, a Diarylpybidine, a Pyridooxazinone, an Indolecarboxamide, a Benzoimidazole carboxamide, a Pyrazolo[4,3-c]quinoline, an Imidazolylquinoline-carbxaldehyde semicarbazide, a Pyridyl Cyanoguanidine, IkB Kinase Inhibitor Peptide, IKK-2 Inhibitor IV ([5-(p-Fluorophenyl)-2-ureido]thiophene-3-carboxamide), IKK Inhibitor II, Wedelolactone, IKK Inhibitor VII, IKK-2 Inhibitor V (N-(3,5-Bis-trifluoromethylphenyl)-5-chloro-2-hydroxybenzamide), IKK-2 Inhibitor VI (5-Phenyl-2-ureido)thiophene-3-carboxamide), IKK-2 Inhibitor VIII ACHP (2-Amino-6-(2-(cyclopropylmethoxy)-6-hydroxyphenyl)-4-(4-piperidinyl)-3-pyridinecarbonitrile), and their derivatives or analogues. In yet another preferred embodiment, the IKK inhibitor is ML120B or LY2409881.

A further preferred embodiment relates to the use according to this invention, wherein said combination comprises a BCL-2 inhibitor, which is selected from the group consisting of 4-[4-[[2-(4-chlorophenyl)-5,5-dimethyl-1-cyclohexen-1-yl]methyl]-1-piperazinyl]-N-[[4-[[(1R)-3-(4-morpholinyl)-1-[(phenylthio)methyl]propyl]amino]-3-[(trifluoromethyl)sulfonyl]phenyl] sulfonyl]benzamide (navitoclax or ABT-263), tetrocarcin A, antimycin, gossypol (such as (-)-gossypol acetic acid), obatoclax (GX15-070), ethyl 2-amino-6-bromo-4-(1-cyano-2-ethoxy-2-oxoethyl)-4H-chromene-3-carboxylate (HA 14-1), oblimersen, a Bak BH3 peptide, 4-[4-[(4'-chloro[1,1'-biphenyl]-2-yl)methyl]-1-piperazinyl]-N-[[4-[[(1R)-3-(dimethylamino)-1-[(phenylthio)methyl]propyl]amino]-3-nitrophenyl]sulfonyl]-benzamide (ABT-737), 2-((1H-pyrrolo[2,3-b]pyridin-5-yl)oxy)-4-(44(4'-chloro-5,5-dimethyl-3,4,5,6-tetrahydro-[1,1'-biphenyl]-2-Amethyl)piperazin-1-yl)-N-((3-nitro-4-(((tetrahydro-2H-pyran-4-Amethyl)amino)phenyl)sulfonyl)benzamide (venetoclax), S55746 (BCL201), and their derivatives or analogues. In a particularly preferred embodiment, the BCL-2 inhibitor is venetoclax.

A particularly preferred embodiment of this invention pertains to the use of
**(a)** A RIPK1 inhibitor;
**(b)** An IKK/NFκB - signaling inhibitor; and
**(c)** A BCL-2 inhibitor,
for the manufacture of a medicament for the treatment and/or prevention of a proliferative disease, wherein the RIPK1 inhibitor (a) is Nec-is, the IKK/NFκB - signaling inhibitor (b) is ML120B or LY2409881, and the BCL-2 inhibitor (c) is venetoclax, or any derivative, analogue, or salt of these compounds.

A further preferred embodiment relates to the use of
**(a)** A RIPK1 inhibitor;
**(b)** An IKK/NFκB - signaling inhibitor; and
**(c)** A BCL-2 inhibitor,
for the manufacture of a medicament for the treatment and/or prevention of a proliferative disease, wherein the proliferative disease is preferably a solid cancer or a hematologic cancer, for example including leukemias, lymphomas, and myelomas.

Further preferred is the use of
**(a)** A RIPK1 inhibitor;
**(b)** An IKK/NFκB - signaling inhibitor; and
**(c)** A BCL-2 inhibitor,
for the manufacture of a medicament for the treatment and/or prevention of a proliferative disease, wherein the proliferative disease is a cancer selected from B cell non-Hodgkin's lymphoma (B-NHL), T cell non-Hodgkin's lymphoma (T-NHL), acute myeloid leukemia (AML), acute lymphoblastic leukemia (ALL), such as B-cell ALL or T-cell ALL, for example precursor B-cell ALL, precursor T-cell ALL, mature B-cell ALL, or mature T-cell ALL, chronic lymphocytic leukemia (CLL), small lymphocytic lymphoma (SLL), Hodgkin's lymphoma, multiple myeloma, myelodysplastic syndrome (MDS), mantle-cell lymphoma (MCL), diffuse large B-cell lymphoma, activated B cell type diffuse large B cell lymphoma (ABC-DLBCL), cluster 1 diffuse large B cell lymphoma, and cluster 5 diffuse large B cell lymphoma; and preferably is B cell non-Hodgkin's lymphoma (B-NHL) and/or B-cell mature acute lymphoblastic leukemia (B-cell ALL).

The herein disclosed compounds, combinations and compositions are in particular useful in a method of preventing or treating a subject suffering from a proliferative disease such as cancer, the method comprising the administration of a therapeutically effective amount of a RIPK1 inhibitor, an IKK/NFκB - signaling inhibitor, and a BCL-2 inhibitor to the subject.

In yet another aspect of this invention, which can be combined with any one of the other aspects and/or embodiments of this invention, there is provided a method for the treatment and/or prevention of a proliferative disease in a subject, the method comprising one or more steps of administering to the subject as single treatments or one or more combinatorial treatments, either sequentially or concomitantly, a therapeutically effective amount of compounds (a) to (c):
**(a)** A Receptor-interacting serine/threonine-protein kinase 1 (RIPK1) inhibitor;
**(b)** An Inhibitor of κB (IκB) Kinase (IKK)/Nuclear Factor κB (NFκB) - signaling inhibitor; and
**(c)** A B-cell lymphoma 2 (BCL-2) inhibitor.

Treatment or prevention in some embodiments comprises the administration of a therapeutically effective amount of a RIPK1 inhibitor, an IKK/NFκB - signaling inhibitor, and a BCL-2 inhibitor to the subject. Such an administration may be a sequential or concomitant administration of a therapeutically effective amount of a RIPK1 inhibitor, an IKK/NFκB signaling inhibitor, and a BCL-2 inhibitor to the subject.

As used herein, the term "therapeutically effective amount" means that amount of a compound or combination that will elicit the biological or medical response of a tissue, system, animal or human subject that is being sought, for instance, by a researcher or clinician, in accordance with the herein disclosed invention. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder.

In accordance with all aspects and embodiments of the medical uses and methods of treatment provided herein, the effective amount of the compounds or combinations administered at least once to a subject in need of treatment with a RIPK1 inhibitor, an IKK/NFκB - signaling inhibitor, and a BCL-2 inhibitor is, typically, between about 0.01 mg/kg and about 100 mg/kg per administration, such as between about 1 mg/kg and about 10 mg/kg per administration. In some embodiments, the effective amount administered at least once to said subject of a RIPK1 inhibitor, an IKK/NFκB - signaling inhibitor, and a BCL-2 inhibitor is between about 0.01 mg/kg and about 0.1 mg/kg per administration, between about 0.1 mg/kg and about 1 mg/kg per administration, between about 1 mg/kg and about 5 mg/kg per administration, between about 5 mg/kg and about 10 mg/kg per administration, between about 10 mg/kg and about 50 mg/kg per administration, or between about 50 mg/kg and about 100 mg/kg per administration.

For the prevention or treatment of a disease according to this invention, the appropriate dosage of a RIPK1 inhibitor, an IKK/NFκB - signaling inhibitor, and a BCL-2 inhibitor (or a pharmaceutical composition comprised thereof) will depend on the type of disease to be treated, the severity and course of the disease, whether the RIPK1 inhibitor, an IKK/NFκB - signaling inhibitor, and a BCL-2 inhibitor and/or pharmaceutical composition is administered for preventive or therapeutic purposes, previous therapy, the patient's clinical history, age, size/weight and response to the RIPK1 inhibitor, the IKK/NFκB - signaling inhibitor, and the BCL-2 inhibitor and/or pharmaceutical composition, and the decision of the attending physician. The RIPK1 inhibitor, IKK/NFκB - signaling inhibitor, and BCL-2 inhibitor and/or pharmaceutical composition is suitably administered to the patient at one time or over a series of treatments. If such RIPK1 inhibitor, IKK/NFκB - signaling inhibitor, and BCL-2 inhibitor and/or pharmaceutical composition is administered over a series of treatments, the total number of administrations for a given course of treatment may consist of a total of about 2, 3, 4, 5, 6, 7, 8, 9, 10 or more than about 10 treatments. For example, a treatment may be given once every day (or 2, 3 or 4 times a day) for a week, a month or even several months. In certain embodiments, the course of treatment may continue indefinitely.

A particularly preferred embodiment pertains to the method for the treatment and/or prevention of a proliferative disease in a subject, the method comprising any of the following treatment regimens of administrations to the subject:
(i) Administration of a therapeutically effective amount of a therapeutic composition comprising compounds (a) to (c);
(ii) Administration of a therapeutically effective amount of a first, second and third therapeutic composition comprising each independently one of compounds (a) to (c), and wherein all three compositions together comprise all of (a) to (c);
(iii) Administration of a therapeutically effective amount of a first therapeutic composition comprising any two of compounds (a) to (c), such as (a)+(b), (a)+(c), or (b)+(c), and administration of a therapeutically effective amount of a second therapeutic composition comprising the compound (a) to (c) not comprised in the first therapeutic composition.

Further preferred is that in the cases of treatment regimens (ii) or (iii) as described above, the first, second and/or third therapeutic compositions are administered to the subject concomitantly or sequentially, preferably in non-overlapping temporal intervals, for example in the order of administering the first therapeutic composition and after a to-be-determined time interval administering to the subject the second and/or third therapeutic composition, and optionally wherein the second and third therapeutic composition are administered in non-overlapping temporal intervals, for example administering the second therapeutic composition and after a to-be-determined time interval administering to the subject the third therapeutic composition.

In a further preferred embodiment of the present invention, any one of the RIPK1 inhibitor (a), the IKK/NFκB - signaling inhibitor, (b), and the BCL-2 inhibitor (c) are selected from a small molecule, a polypeptide, peptide, glycoprotein, a peptide-mimetic, an antigen binding protein (ABP) (for example, an antibody, antibody-like molecule or other antigen binding derivative, or an or antigen binding fragment thereof), a nucleic acid such as a DNA or RNA, for example an antisense or inhibitory DNA or RNA, a ribozyme, an RNA or DNA aptamer, RNAi, siRNA, shRNA and the like, including variants or derivatives thereof such as a peptide nucleic acid (PNA), a genetic construct for targeted gene editing, such as a CRISPR/Cas9 construct and/or a guide nucleic acid (gRNA or gDNA) and/or tracrRNA.

A particularly preferred embodiment pertains to the method the treatment and/or prevention of a proliferative disease in a subject, wherein the RIPK1 inhibitor is selected from the group consisting of necrostatin-1 stable (5-((7-chloro-1H-indol-3-yl)methyl)-3-methyl-2,4-imidazolidinedione), necrostatin-1 (5-(1H-indo1-3-ylmethyl)-3-methyl-2-thioxo-4-imidazolidinone), necrostatin-2 (5-[(7-chloro-1H-indol-3-yl)methyl]-3-methyl-2,4-imidazolidinedione), necrostatin-3(3-phenyl-3,3a,4,5-tetrahydro-2H-benz[g]indazole, 3-phenyl-2,3,3a,4-tetrahydro[1]benzopyrano[4,3-c]pyrazole, 3-phenyl-2,3,3a,4-tetrahydro[1]benzothiopyrano[4,3-c]pyrazole, 5,5-dioxo-3-phenyl-2,3,3a,4-tetrahydro[1]benzothiopyrano[4,3-c]pyrazole]), necrostatin-4((S)-N-(1-[2-chloro-6-fluorophenyl]ethyl)-5-cyano-1-methyl-1H-pyrrole-2-carboxamide), necrostatin-5(2-[[3,4,5,6,7,8-hexahydro-3-(4-methoxyphenyl)-4-oxo[1]benzothieno[2,3-d]pyrimidin-2-yl]thio]-acetonitrile), necrostatin-7(5-((3-(4-fluorophenyl)-1H-pyrazol-4-yl)methylene)-2-imino-3-(thiazol-2-yl)thiazolidin-4-one), necrostatin-21, (S)-5-benzyl-N-(7,9difluoro-2-oxo-2,3,4,5-tetrahydro-1H-benzo[b]azepin-3-yl)1H-1,2,4-triazole-3-carboxamide (GSK3145095), N-benzyl-N-hydroxy-2,2-dimethylbutanamide (RIPA-56), DNL747 (SAR 443060), (*S*)-*N*-(5-methyl-4-oxo-2,3,4,5-tetrahydrobenzo[*b*][1,4]oxazepin-3-yl)-4,5,6,7-tetrahydro-1*H*-indazole-3-carboxamide, (*S*)-*N*-(5-methyl-4-oxo-2,3,4,5-tetrahydrobenzo[*b*][1,4]oxazepin-3-yl)-4*H*-1,2,4-triazole-3-carboxamide, (*R*)-5-methyl-*N*-((*S*)-5-methyl-4-oxo-2,3,4,5-tetrahydrobenzo[*b*][1,4]oxazepin-3-yl)-4,5,6,7-tetrahydro-1H-indazole-3-carboxamide, (*R*)-5-methyl-*N*-((*S*)-5-methyl-4-oxo-2,3,4,5-tetrahydrobenzo[*b*][1,4]oxazepin-3-yl)-4,5,6,7-tetrahydro-1H-indazole-3-carboxamide, (S)-6,6-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (S)-N-(5-methyl-4-oxo-2,3,4,5-tetrahydrobenzo[b][1,4]oxazepin-3-yl)-1',4',5',7'-tetrahydrospiro[cyclopropane-1,6'-indazole]-3'-carboxamide, N-((S)-5-methyl-4-oxo-2,3,4,5-tetrahydrobenzo[b][1,4]oxazepin-3-yl)-1,4,4a,5,5a,6-hexahydrocyclopropa[f]indazole-3-carboxamide, N-((S)-5-methyl-4-oxo-2,3,4,5-tetrahydrobenzo[b][1,4]oxazepin-3-yl)-3,4,5,5a,6,6a-hexahydrocyclopropa[e]indazole-1-carboxamide, (S)-5,5-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (R)-5-(tert-butyl)-N-((S)-5-methyl-4-oxo-2,3,4,5-tetrahydrobenzo[b][1,4]oxazepin-3-yl)-4,5,6,7-tetrahydro-1H-indazole-3-carboxamide, N-((S)-5-methyl-4-oxo-2,3,4,5-tetrahydrobenzo[b][1,4]oxazepin-3-yl)-5-phenyl-4,5,6,7- tetrahydro-1H-indazole-3-carboxamide, N-((S)-5-methyl-4-oxo-2,3,4,5-tetrahydrobenzo[b][1,4]oxazepin-3-yl)-5-(1H-pyrazol-1-yl)-4,5,6,7-tetrahydro-1H-indazole-3-carboxamide, (R)-1-methyl-N-((S)-5-methyl-4-oxo-2,3,4,5-tetrahydrobenzo[b][1,4]oxazepin-3-yl)-5-(trifluoromethyl)-4,5,6,7-tetrahydro-1H-indazole-3-carboxamide, (S)-N-(5-methyl-4-oxo-2,3,4,5-tetrahydrobenzo[b][1,4]oxazepin-3-yl)-5-(trifluoromethyl)-1H-indazole-3-carboxamide, (S)-N-(5-methyl-4-oxo-2,3,4,5-tetrahydrobenzo[b][1,4]oxazepin-3-yl)-5-(trifluoromethyl)-1H-pyrazolo[3,4-b]pyridine-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-[1,2,3]triazolo [1,5-a]pyridine-3-carboxamide, (S)-N-(5-methyl-4-oxo-2,3,4,5-tetrahydrobenzo[b][1,4]oxazepin-3-yl)-7-(trifluoromethyl)imidazo[1,5-a]pyridine-1-carboxamide, (S)-N-(5-methyl-4-oxo-2,3,4,5-tetrahydrobenzo[b][1,4]oxazepin-3-yl)-6-(trifluoromethyl)-[1,2,4]triazolo[4,3-b]pyridazine-3-carboxamide, (S)-5-isopropyl-N-(5-methyl-4-oxo-2,3,4,5-tetrahydrobenzo[b][1,4]oxazepin-3-yl)-1H-pyrazolo[3,4-b]pyridine-3-carboxamide, (S)-N-(5-methyl-4-oxo-2,3,4,5-tetrahydrobenzo[b][1,4]oxazepin-3-yl)-5-(perfluoroethyl)-1H-pyrazolo[3,4-b]pyridine-3-carboxamide, N-((S)-5-methyl-4-oxo-2,3,4,5-tetrahydrobenzo[b][1,4]oxazepin-3-yl)-5-(perfluoroethyl)-4,5,6,7-tetrahydro-[1,2,3]triazolo[1,5-a]pyridine-3-carboxamide, (S)-N-(5-methyl-4-oxo-2,3,4,5-tetrahydrobenzo[b][1,4]oxazepin-3-yl)-6-(perfluoroethyl)-[1,2,4]triazolo[4,3-b]pyridazine-3-carboxamide, (S)-5-benzyl-N-(5-methyl-4-oxo-2,3,4,5-tetrahydrobenzo[b][1,4]oxazepin-3-yl)isoxazole-3-carboxamide (GSK481), 1-[(5S)-4,5-Dihydro-5-phenyl-1H-pyrazol-1-yl]-2,2-dimethyl-1-propanone, 2,2-Dimethyl-1-(5(S)-phenyl-4,5-dihydro-pyrazol-1-yl)-propan-1-one (GSK963), (S)-5-benzyl-N-(5-methyl-4-oxo-2,3,4,5-tetrahydrobenzo[b][1,4]oxazepin-3-yl)-1H-1,2,4-triazole-3-carboxamide (GSK2982772), 5-(indol-3-ylmethyl)-3-methyl-2-thio-hydantoin), and their derivatives or analogues; and preferably is necrostatin-1 stable (Nec-1s).

In a further preferred embodiment, the IKK/NFκB signaling inhibitor is an IKK inhibitor, preferably an IKK2/IKKβ inhibitor. Further preferred is that the IKK2/IKKβ inhibitor is selected from the group consisting of N-(6-chloro-7-methoxy-9H-beta-carbolin-8-yl)-2-methylnicotinamide (ML120B), LY2409881, (3S)-N-(6-Chloro-9H-beta-carbolin-8-yl)-4-(2-((2R,6S)-2,6-dimethylmorpholin-4-yl)-2-oxoethyl)-6,6-dimethylmorpholine-3-carboxamide (MLN-0415), 2-(pyrimidin-4-yl)-1H-indole (SAR-113945), 1-(5-Methoxy-2-thiophen-2-yl-quinazolin-4-ylamino)-3-methyl-pyrrole-2,5-dione (CDC-839), 1H-imidazo[4,5-b]pyridin-5-amine, 7-[5-[(cyclohexylmethylamino)-methyl]-1H-indol-2-yl]-2-methyl, sulfate trihydrate (E-6070), TPCA-1, Bardoxolone Methyl, Resveratrol, MRT67307, AmLexanox, BMS-345541, IKK 16, BI605906, ACHP Hydrochloride, PS-1145, Apigenin, SC-514, IKKε-IN-1, IMD-0354, AZD 3264, Icariin (Ieariline), Bay 65-1942 Hcl, Bay 65-1942, an Anilino-Pyrimidine, IKK inhibitor III, SC-514*Amino-imidazolecarboxamide, an Ureudo-thiophenecarboxamide, a Diarylpybidine, a Pyridooxazinone, an Indolecarboxamide, a Benzoimidazole carboxamide, a Pyrazolo[4,3-c]quinoline, an Imidazolylquinoline-carbxaldehyde semicarbazide, a Pyridyl Cyanoguanidine, IkB Kinase Inhibitor Peptide, IKK-2 Inhibitor IV ([5-(p-Fluorophenyl)-2-ureido]thiophene-3-carboxamide), IKK Inhibitor II, Wedelolactone, IKK Inhibitor VII, IKK-2 Inhibitor V (N-(3,5-Bis-trifluoromethylphenyl)-5-chloro-2-hydroxybenzamide), IKK-2 Inhibitor VI (5-Phenyl-2-ureido)thiophene-3-carboxamide), IKK-2 Inhibitor VIII ACHP (2-Amino-6-(2-(cyclopropylmethoxy)-6-hydroxyphenyl)-4-(4-piperidinyl)-3-pyridinecarbonitrile), and their derivatives or analogues; and preferably is ML120B or LY2409881.

Another preferred embodiment pertains to the method of treatment and/or prevention of a proliferative disease in a subject, wherein the BCL-2 inhibitor is selected from the group consisting of 4-14-1[2-(4-chlorophenyl)-5,5-dimethyl-1-cyclohexen-1-yl]methyl]-1-piperazinyl]-N-[[4-[[(1R)-3-(4-morpholinyl)-1-[(phenylthio)methyl]propyl]amino]-3-[(trifluoromethyl)sulfo nyl]phenyl] sulfonyl]benzamide (navitoclax or ABT-263), tetrocarcin A, antimycin, gossypol (such as (-)-gossypol acetic acid), obatoclax (GX15-070), ethyl 2-amino-6-bromo-4-(1-cyano-2-ethoxy-2-oxoethyl)-4H-chromene-3-carboxylate (HA 14-1), oblimersen, a Bak BH3 peptide, 4-[4-[(4'-chloro[1,1'-biphenyl]-2-yl)methyl]-1-piperazinyl]-N-[[4-[[(1R)-3-(dimethylamino)-1-[(phenylthio)methyl]propyl]amino]-3-nitrophenyl]sulfonyl]-benzamide (ABT-737), 2-((1H-pyrrolo[2,3-b]pyridin-5-yl)oxy)-4-(44(4'-chloro-5,5-dimethyl-3,4,5,6-tetrahydro-[1,1'-biphenyl]-2-Amethyl)piperazin-1-yl)-N-((3-nitro-4-(((tetrahydro-2H-pyran-4-Amethyl)amino)phenyl)sulfonyl)benzamide (venetoclax), S55746 (BCL201), and their derivatives or analogues; and preferably is venetoclax.

Yet another embodiment of this invention pertains to a method for the treatment and/or prevention of proliferative disease in a subject, the method comprising one or more steps of administering to the subject as single treatments or one or more combinatorial treatments, either sequentially or concomitantly, a therapeutically effective amount of compounds (a) to (c):
**(a)** A RIPK1 inhibitor;
**(b)** An IKK/ NFκB - signaling inhibitor; and
**(c)** A BCL-2 inhibitor,
wherein (a) is Nec-is, (b) is ML120B or LY2409881, and (c) is venetoclax, or any derivative, analogue, or salt of these compounds.

A further embodiment of this invention relates to a method for the treatment and/or prevention of a proliferative disease in a subject, wherein the proliferative disease is preferably a solid cancer or a hematologic cancer, for example including leukemias, lymphomas, and myelomas.

Particularly preferred is a method for the treatment and/or prevention of a proliferative disease in a subject, wherein the proliferative disease is a cancer selected from B cell non-Hodgkin's lymphoma (B-NHL), T cell non-Hodgkin's lymphoma (T-NHL), acute myeloid leukemia (AML), acute lymphoblastic leukemia (ALL), such as B-cell ALL or T-cell ALL, for example precursor B-cell ALL, precursor T-cell ALL, mature B-cell ALL, or mature T-cell ALL, chronic lymphocytic leukemia (CLL), small lymphocytic lymphoma (SLL), Hodgkin's lymphoma, multiple myeloma, myelodysplastic syndrome (MDS), mantle-cell lymphoma (MCL), diffuse large B-cell lymphoma, activated B cell type diffuse large B cell lymphoma (ABC-DLBCL), cluster 1 diffuse large B cell lymphoma, and cluster 5 diffuse large B cell lymphoma; and preferably is B cell non-Hodgkin's lymphoma (B-NHL) and/or B-cell mature acute lymphoblastic leukemia (B-cell ALL).

In all aspects of this invention, the subject is preferably a mammal, such as a mouse, rat, guinea pig, rabbit, cat, dog, monkey, or preferably a human, for example a human patient, more preferably a human patient suffering from a proliferative disease.

Yet another particularly preferred aspect relates to the method for the treatment and/or prevention of a proliferative disease in a subject, wherein said therapeutically effective amount of compounds (a) to (c) is administered to said subject by intravenous, vaginal, oral, intranasal, intrathecal, intra-arterial, intradermal, subcutaneous, transdermal (topical), intracerebroventricular, intraparenchymal, intratumoral, transmucosal, rectal, bronchial, and/or parenteral administration, or by any clinically/medically accepted method.

Additionally preferred is that said therapeutically effective amount of compounds (a) to (c) is provided in form of a tablet, a pill, a capsule, a troche, a dragee, a powder, an aerosol spray, a nasal spray, a suppository, a gel, an ointment, a salve, a cream, and/or a solution.

Finally, in a further aspect of this invention, which can be combined with any of the other aspects and any of the particularly preferred embodiments, the present invention provides a kit comprising:
i) a therapeutically effective amount of a compound for use according to this invention, a pharmaceutical composition according to this invention, or a combination for use according to this invention,
ii) written instructions to apply said therapeutically effective amount of said compound for use, said pharmaceutical composition, or said combination for use to a subject; and
iii) optionally, a container holding said compound, said pharmaceutical composition, or said combination for use and the written instructions.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

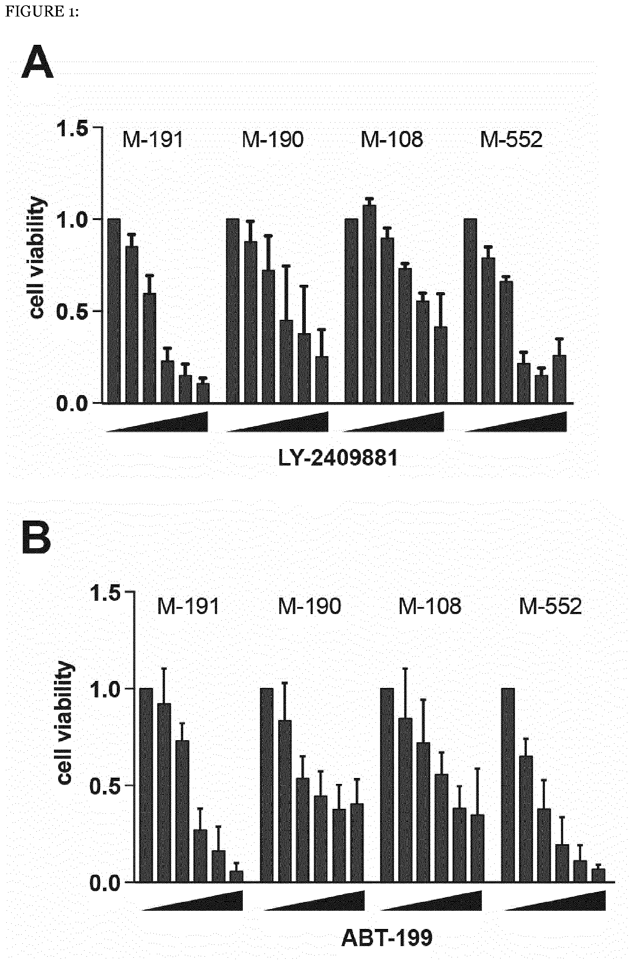

**Figure 1** **shows the pharmacological inhibition potential of IKK2 and BCL-2 to kill lymphoma cells.** The murine MBC lines M-191, M-190, M-108 and M-552 were treated with (A) the IKK2 inhibitor LY-2409881 (0, 0.185, 0.556, 1.667, 5, 15 µM) or (B) the BCL-2 inhibitor ABT-199 (0, 11.1, 33.3, 100, 300 nM) for 72 hours and cell viability was measured by CellTiter-Glo ^{®}. The illustrations in Fig. 1 represent three independent experiments.

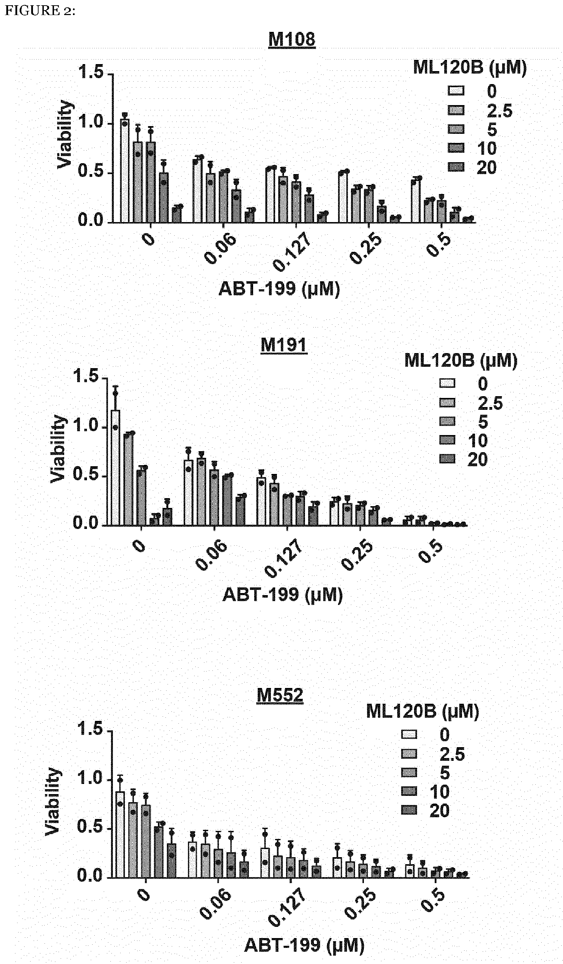

**Figure 2** **shows the pharmacological inhibition of IKK2 strongly synergizes with BCL-2 inhibition to kill lymphoma cells.** The murine MBC lines M-191, M-190, M-108 and M-552 were treated with ABT-199 and/or IKK2-inhibitor ML120B at the indicated doses for 72 hours and cell viability was measured by CellTiter-Glo ^{®}. The illustrations in Fig. 2 represent two independent experiments.

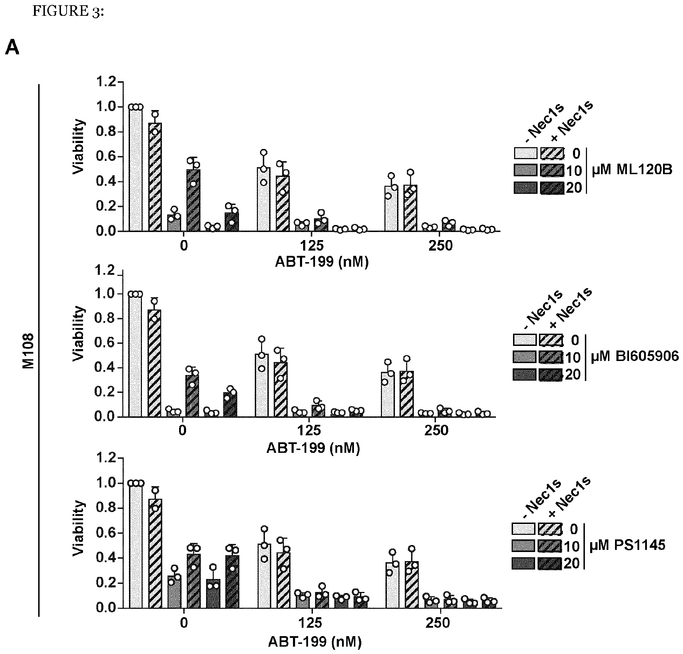

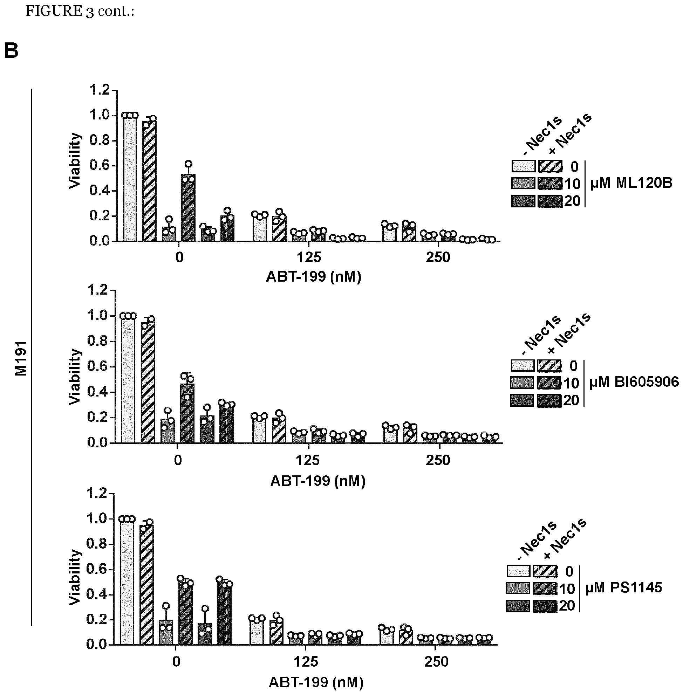

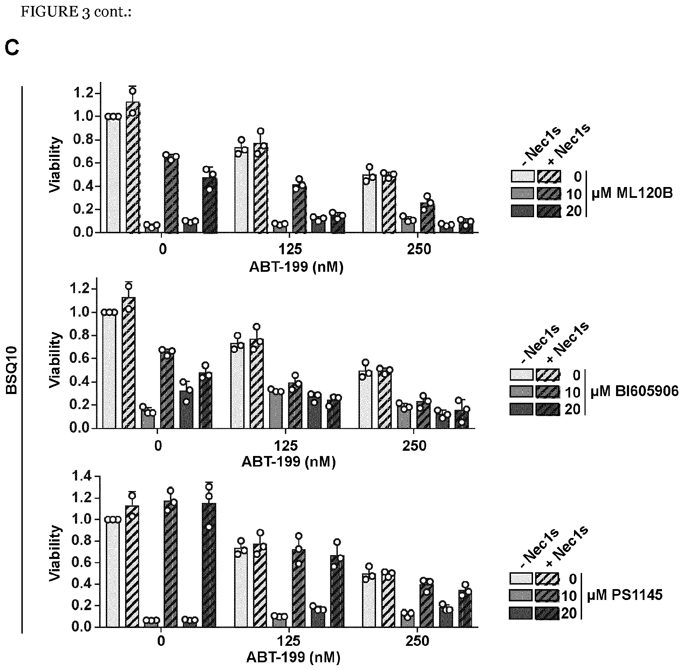

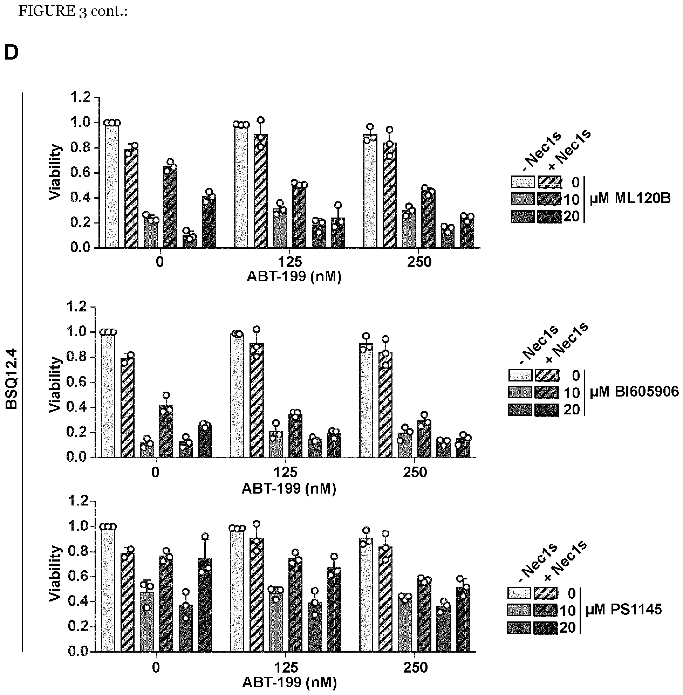

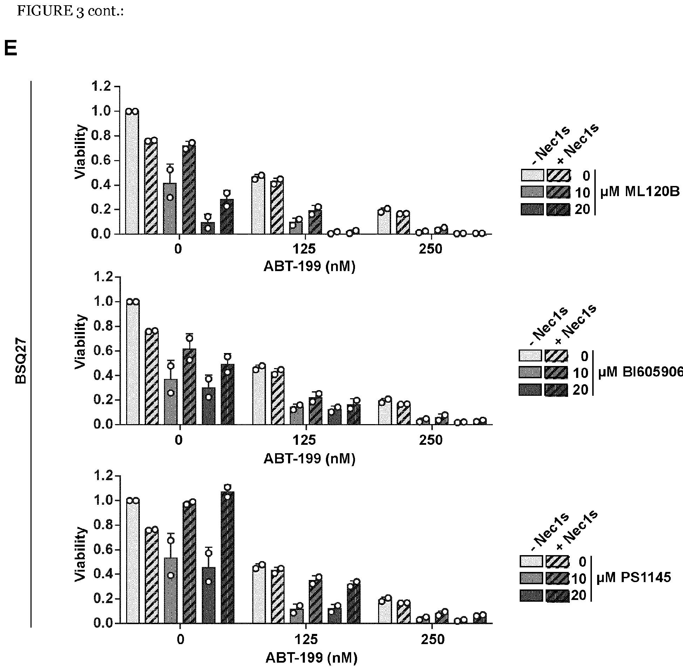

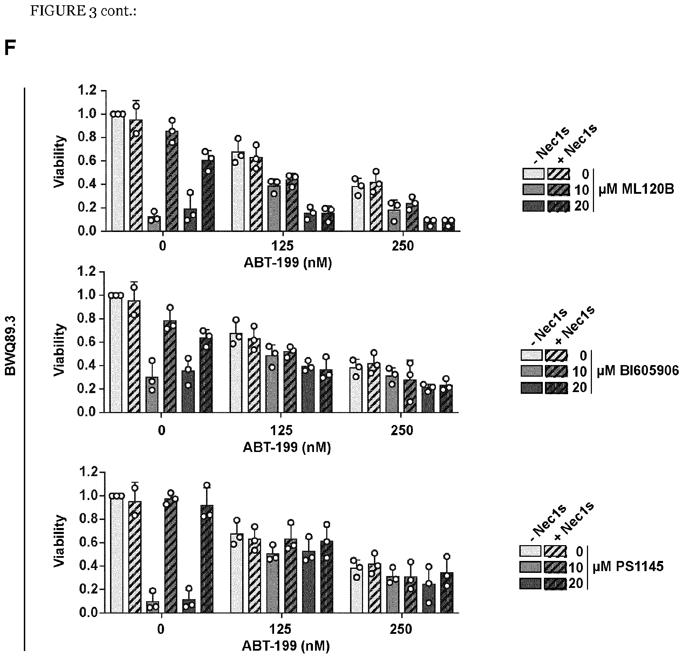

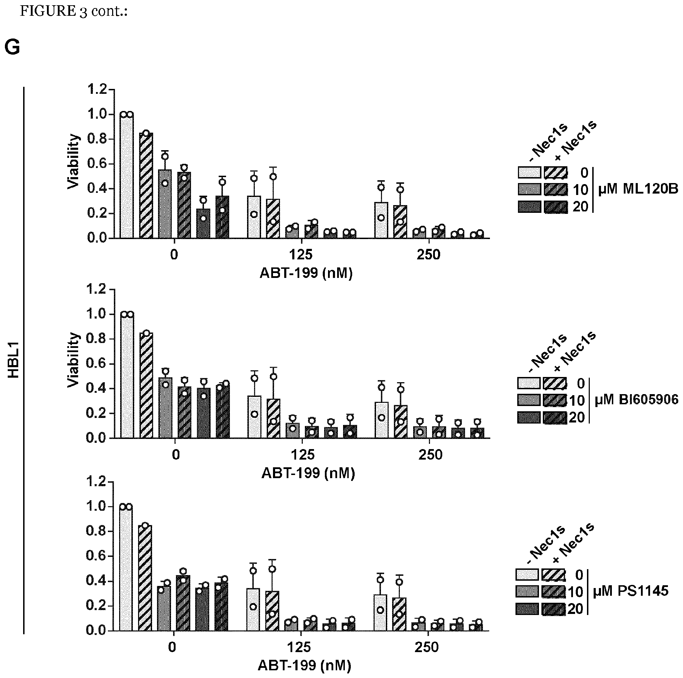

**Figure 3** **shows the combined pharmacological inhibition of IKK2 and RIPK1 strongly synergizes with BCL-2 inhibition to kill lymphoma cells.** Murine (A-F) and human (G) cell lines were treated with IKK2 inhibitors in the absence or presence of a RIPK1 inhibitor and/or a BCL-2 inhibitor as indicated. Cell viability was measured after 72 hrs using CellTiterGlo assay. The data points represent mean values of independent experiments. A. The murine cell line M108 was treated with the IKK2 inhibitors ML120B, BI605906 and PS1145 in the absence or presence of the RIPK1 inhibitor Necis (10 µM or 20 µM, as indicated) and/or the BCL-2 inhibitor ABT-199 (125 nM or 250 nM as indicated). B. The murine cell line M191 was treated with the IKK2 inhibitors ML120B, PS1145 and BI605906 in the absence or presence of the RIPK1 inhibitor Necis (10 µM or 20 µM, as indicated) and/or the BCL-2 inhibitor ABT-199 (125 nM or 250 nM as indicated). C. The murine cell line BSQ10 was treated with the IKK2 inhibitors ML120B, PS1145 and BI605906 in the absence or presence of the RIPK1 inhibitor Necis (10 µM or 20 µM, as indicated) and/or the BCL-2 inhibitor ABT-199 (125 nM or 250 nM as indicated). D. The murine cell line BSQ12.4 was treated with the IKK2 inhibitors ML120B, PS1145 and BI605906 in the absence or presence of the RIPK1 inhibitor Necis (10 µMor 20 µM, as indicated) and/or the BCL-2 inhibitor ABT-199 (125 nM or 250 nM as indicated). E. The murine cell line BSQ27 was treated with the IKK2 inhibitors ML120B, PS1145 and BI605906 in the absence or presence of the RIPK1 inhibitor Necis (10 µM or 20 µM, as indicated) and/or the BCL-2 inhibitor ABT-199 (125 nM or 250 nM as indicated). F. The murine cell line BWQ89.3 was treated with the IKK2 inhibitors ML120B, PS1145 and BI605906 in the absence or presence of the RIPK1 inhibitor Necis (10 µM or 20 µM, as indicated) and/or the BCL-2 inhibitor ABT-199 (125 nM or 250 nM as indicated). G. The human cell line HBL1 was treated with the IKK2 inhibitors ML120B, PS1145 and BI605906 in the absence or presence of the RIPK1 inhibitor Necis (10 µM or 20 µM, as indicated) and/or the BCL-2 inhibitor ABT-199 (125 nM or 250 nM as indicated).

The sequences with SEQ ID NOs. 1 to 3 show:
SEQ ID NO: 1 (RIPK1 isoform 1, human): 
MQPDMSLNVIKMKSSDFLESAELDSGGFGKVSLCFHRTQGLMIMKTVYKGPNCIEHNEALLEE
AKMMNRLRHSRVVKLLGVIIEEGKYSLVMEYMEKGNLMHVLKAEMSTPLSVKGRIILEIIEGM
CYLHGKGVIHKDLKPENILVDNDFHIKIADLGLASFKMWSKLNNEEHNELREVDGTAKKNGG
TLYYMAPEHLNDVNAKPTEKSDVYSFAVVLWAIFANKEPYENAICEQQLIMCIKSGNRPDVDDI
TEYCPREIISLMKLCWEANPEARPTFPGIEEKFRPFYLSQLEESVEEDVKSLKKEYSNENAVVKR
MQSLQLDCVAVPSSRSNSATEQPGSLHSSQGLGMGPVEESWFAPSLEHPQEENEPSLQSKLQD
EANYHLYGSRMDRQTKQQPRQNVAYNREEERRRRVSHDPFAQQRPYENFQNTEGKGTAYSSA
ASHGNAVHQPSGLTSQPQVLYQNNGLYSSHGFGTRPLDPGTAGPRVWYRPIPSHMPSLHNIPV
PETNYLGNTPTMPFSSLPPTDESIKYTIYNSTGIQIGAYNYMEIGGTSSSLLDSTNTNFKEEPAAK
YQAIFDNTTSLTDKHLDPIRENLGKHWKNCARKLGFTQSQIDEIDHDYERDGLKEKVYQMLQK
WVMREGIKGATVGKLAQALHQCSRIDLLSSLIYVSQN
SEQ ID NO: 2 (IKK2 isoform 1, human):
SEQ ID NO: 3 (BCL-2, human):

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Pharmacological inhibition potential of IKK2 and BCL-2 to kill lymphoma cells

The murine MBC M-191, M-190, M-108, and M-552 cell lines were generated by adopting cells from murine Myd88p.L252P/wt;R26LSL.BCL2/wt;CD19Cre/wt tumors to cell culture conditions. BSQ10, BSQ12.4 and BSQ27 are stable cell lines generated from Cd79bp.Y195H/wt;Myd88p.L252P/wt;R26LSL.BCL2/wt;CD19Cre/wt tumors. BWQ89.3 is a stable cell line derived from a murine Prdm1flox/flox;Myd88p.L252P/wt;R26LSL.BCL2/wt;CD19Cre/wt tumor. The MBC cell lines are derived from tumors spontaneously developing in mice that B cell-specifically express the Myd88 p.L252P mutation (the murine orthologue of the human p.L265P) from the endogenous locus in combination with a strong BCL2 overexpression derived from a Rosa26 knock-in allele. Both MYD88 mutations and BCL2 overexpression are frequently found in ABC-DLBCL and Myd88p. L252P/wt;R26LSL.BCL2/wt;CD19Cre/wt animals (abbreviated MBC) develop an ABC-DLBCL-like disease with high NF-kB activation (17).

To test the pharmacological inhibition potential of IKK2 and BCL-2 to kill lymphoma cells, MBC M-191, M-190, M-108, and M-552 cell lines were cultured in DMEM media (Gibco) containing 10% FCS, 1% penicillin/streptavidin (Gibco), 6 ml L-glutamine (200 mM, Gibco), 6 ml sodium pyruvate (100 mM, Gibco), 6 ml HEPES (1 M), 6 ml non-essential amino acids (100X, Gibco), 50 pM 2-mercaptoethanol. Then, cell viability assays were used to test the pharmacological inhibition potential of IKK2 (using the inhibitor LY2409881) and BCL-2 (using the inhibitor ABT-199) to kill the MBC M-191, M-190, M-108, and M-552 cell lines. Compounds were purchased from the following vendors: ML120B, MedChem Express (HY-15473); PS1145, MedChem Express (HY-18008); BI605906, MedChem Express (HY-13019); Nec1s, BioVision (2263); ABT-199, Medchem Express (HY-15531). All compounds were dissolved in DMSO (ML120B, PS1145, BI605906, Nec1s, 10mM, ABT-199 0.5 mM). Cells were plated in opaque 96-well (Fig. 1) or 384-well (Fig. 2) plates at a density of 70.000 cells/ml (Fig. 1 and 2) and treated with the indicated compounds dissolved in DMSO using a Tecan D300e digital dispenser. Cells were then incubated for 72 hours at 37°C, 5% CO2. Multiwell plates were allowed to equlibrate to room temperature before 30 µl of a CellTiter-Glo^{®} reagent (Promega) volume equal to the culture volume was added to each well. After an incubation time of 15 minutes, the luminescence signal was read on a plate reader (Tecan). All conditions were measured in triplicate, randomized across the plate and the DMSO concentration in each well was adjusted to maximum DMSO concentration (0.4%) in all wells, and normalized to untreated conditions.

The pharmacological inhibition of the central NF-kB-activating kinase IKK2/IKKb, which operates downstream of the BCR and TLR pathways, as shown for the IKK inhibitor LY2409881, is extremely effective in eradicating murine ABC-DLBCL cell lines in vitro (Fig. 1A). Also, BCL2 inhibition, as shown for the BCL-2 inhibitor ABT-199 (also referred to as Venetoclax), displays strong toxicity in these murine ABC-DLBCL cell lines (Fig. 1 B).

### Example 2: BCL-2 inhibition and IKK inhibition synergize to cause increased death of cancer cells

The murine MBC cell lines M-108, M-191, and M-552 were cultured in DMEM media (Gibco) containing 10% FCS, 1% penicillin/streptavidin (Gibco), 6 ml L-glutamine (200 mM, Gibco), 6 ml sodium pyruvate (100 mM, Gibco), 6 ml HEPES (1 M), 6 ml non-essential amino acids (100X, Gibco), 50 µM 2-mercaptoethanol. The cells were treated with different concentrations of the IKK2 inhibitor ML120B and different concentrations of the BCL-2 inhibitor ABT-199, as indicated.

In vitro, the BCL-2 inhibitor ABT-199 (also referred to as Venetoclax) and the IKK2 inhibitor ML120B synergize to potentiate killing of cancer cell lines M108, M191 and M552 (Fig. 2). Fig. 2 shows that BCL-2 inhibition and IKK inhibition synergize to cause increased death of cancer cells, i.e. inhibition of NF-kB signaling using IKK-inhibitors potentiates pro-apoptotic activity of BCL-2 inhibitors. Importantly, combining IKK2 and BCL2 inhibition in ABC-DLBCL explains the highly efficacious and surprisingly high combined effect, as the two central oncogenic pathways in this disease are simultaneously blocked. This combined approach can also reduce the impact of acquired ABT-199 (also referred to as Venetoclax) resistance.

### Example 3: Combined pharmacological inhibition of IKK2 and RIPK1 strongly synergizes with BCL-2 inhibition to kill lymphoma cells.

Fig. 3 shows that the BCL-2 inhibitor ABT-199 and the IKK2 inhibitors ML120B, BI605906 and PS1145 synergize to potentiate killing of murine (M108, M191, BSQ10, BSQ12.4, BSQ27, BWQ89.3) and human (HBL1) cancer cell lines. An increased production of IL-1 beta by myeloid cells treated with, e.g., IKK inhibitors, such as ML120B, depends largely on RIPK1 kinase activity. Accordingly, inhibition of RIPK1 kinase activity either genetically (e.g., using knock-in mice expressing kinase-inactive RIPK1 D138N) or pharmacologically (e.g., using Necrostatin-1, a chemical inhibitor of RIPK1) can diminish the LPS-induced production of IL-1β by macrophages treated with IKK inhibitors. Moreover, genetic inhibition of RIPK1 kinase activity prevents the development of neutrophilia caused by myeloid cell-specific inhibition of IKK2, as depicted in Figure 5 of WO 2019/110832.

Murine cell lines (Fig. 3 A-F) were cultured in DMEM media (Gibco) containing 10% FCS, 1% penicillin/streptavidin (Gibco), 6 ml L-glutamine (200 mM, Gibco), 6 ml sodium pyruvate (100 mM, Gibco), 6 ml HEPES (1 M), 6 ml non-essential amino acids (100X, Gibco), and 50 µM 2-mercaptoethanol. Cells were plated in opaque 384-well plates at a density of 100.000 cells/ml (30 µl of culture volume) and treated with the indicated compounds dissolved in DMSO using a Tecan D300e digital dispenser. The human DLBCL cell line HBL1 (Fig. 3 G) was cultured in RPMI (Gibco) containing 20% FCS (Gibco) and 1% penicilin/streptavidin (Gibco) and treated with the indicated compounds dissolved in DMSO using a Tecan D300e digital dispenser. Cell viability of murine and human cell lines was measured after 72 hrs using CellTiterGlo assay. The data points represent mean values of independent experiments. Interestingly, the synergizing killing effect of the BCL-2 inhibitor ABT-199 and the IKK2 inhibitors ML120B, BI605906 and PS1145 on murine (M108, M191, BSQ10, BSQ12.4, BSQ27, BWQ89.3) and human (HBL1) cancer cell lines is not prevented by the simultaneous treatment of the cancer cell lines with the RIPK1 - Inhibitor Necis (10 µM or 20 µM, as indicated).

In some tumors, IKK inhibition alone may cause cell death but this often depends on RIPK1 kinase activity, since IKK inhibition sensitizes cells to RIPK1-dependent death. While inhibiting RIPK1 alone did not induce the death of murine (M108, M191, BSQ10, BSQ12.4, BSQ27, BWQ89.3) or human (HBL1) cancer cell lines, the presence of increasing concentrations of the BCL2-inhibitor as indicated could overcome the protective effect of the RIPK1 inhibitor (Fig. 3), suggesting that RIPK1 inhibition does not prevent mitochondrial apoptosis induced by the BCL2 inhibitor. Since an increased production of IL-1 beta by myeloid cells treated with IKK inhibitors largely depends on RIPK1 kinase activity, inhibition of RIPK1 kinase activity strongly diminishes the LPS-induced production of IL-1β by macrophages treated with IKK inhibitors. Thus, the most serious known side effect of IKK inhibitors, namely the increased production of IL-1β by myeloid cells, which can lead to the development of neutrophilia, can be prevented by the inhibition of RIPK1. Hence, Fig. 3 demonstrates the surprising effect that the combined use of BCL-2 inhibition and IKK inhibition synergizes to efficiently cause an increased death of cancer cells, and that the simultaneous or concomitant application of RIPK1 inhibitors prevents toxicity induced by IKK inhibition. Thus, inhibition of NF-kB signaling using IKK-inhibitors potentiates pro-apoptotic activity of BCL-2 inhibitors, while inhibition of RIPK1 prevents IKK-inhibition induced toxicity. Therefore, the presence of a RIPK1 inhibitor does not prevent this synergistic cell death-inducing effect of BCL-2 inhibition and IKK inhibition. Accordingly, the use of (a) a Receptor-interacting serine/threonine-protein kinase 1 (RIPK1) inhibitor; (b) an Inhibitor of κB (IκB) Kinase (IKK)/Nuclear Factor κB (NFκB) - signaling inhibitor; and (c) a B-cell lymphoma 2 (BCL-2) inhibitor is highly beneficial for the treatment of proliferative diseases, such as cancer.

### REFERENCES

The references are:
1. Ashkenazi A, Fairbrother WJ, Leverson JD, Souers AJ. From basic apoptosis discoveries to advanced selective BCL-2 family inhibitors. Nat Rev Drug Discov. 2017;16(4):273-84.
2. Shaffer AL, 3rd, Young RM, Staudt LM. Pathogenesis of human B cell lymphomas. Annu Rev Immunol. 2012;30:565-610.
3. Adams CM, Clark-Garvey S, Porcu P, Eischen CM. Targeting the BcI-2 Family in B Cell Lymphoma. Front Oncol. 2018;8:636.
4. Davids MS. Targeting BCL-2 in B-cell lym-phomas. Blood. 2017;130(9)1081-8. 5. Fischer K, Al-Sawaf 0, Bahlo J, Fink AM, Tandon M, Dixon M, et al. Venetoclax and Obinutuzumab in Patients with CLL and Coexisting Conditions. N Engl J Med. 2019;380(23):2225-36.
6. Souers AJ, Leverson JD, Boghaert ER, Ackler SL, Catron ND, Chen J, et al. Venetoclax, a potent and selective BCL-2 inhibitor, achieves antitumor activity while sparing platelets. Nat Med. 2013;19(2):202-8.
7. Herling CD, Abedpour N, Weiss J, Schmitt A, Jachimowicz RD, Merkel 0, et al. Clonal dynamics towards the development of venetoclax resistance in chronic lymphocytic leukemia. Nat Commun. 2018;9(1):727.
8. Cory S, Roberts AW, Colman PM, Adams JM. Targeting BCL-2-like Proteins to Kill Cancer Cells. Trends Cancer. 2016;2(8):443-60.
9. Gasparini C, Celeghini C, Monasta L, Zauli G. NF-kappaB pathways in hematological malignancies. Cell Mol Life Sci. 2014;71(11):2083-102.
10. Jost PJ, Ruland J. Aberrant NF-kappaB signaling in lymphoma: mechanisms, consequences, and therapeutic implications. Blood. 2007;109(7):2700-7.
11. Rahal R, Frick M, Romero R, Korn JM, Kridel R, Chan FC, et al. Pharmacological and genomic profiling identifies NF-kappaB-targeted treatment strategies for mantle cell lymphoma. Nat Med. 2014;20(1):87-92.
12. Davis RE, Ngo VN, Lenz G, Tolar P, Young RM, Romesser PB, et al. Chronic active B-cell-receptor signaling in diffuse large B-cell lymphoma. Nature. 2010;463(7277):88-92.
13. Ngo VN, Davis RE, Lamy L, Yu X, Zhao H, Lenz G, et al. A loss-of-function RNA interference screen for molecular targets in cancer. Nature. 2006;441(7089):106-10.
14. Deng C, Lipstein M, Rodriguez R, Serrano XO, McIntosh C, Tsai WY, et al. The novel IKK2 inhibitor LY2409881 potently synergizes with histone deacetylase inhibitors in preclinical models of lymphoma through the downregulation of NF-kappaB. Clin Cancer Res. 2015;21(1):134-45.
15. Catz SD, Johnson JL. Transcriptional regulation of bcl-2 by nuclear factor kappa B and its significance in prostate cancer. Oncogene. 2001;20(50):7342-51.
16. Hinz M, Loser P, Mathas S, Krappmann D, Dorken B, Scheidereit C. Constitutive NF-kappaB maintains high expression of a characteristic gene network, including CD40, CD86, and a set of antiapoptotic genes in Hodgkin/Reed-Sternberg cells. Blood. 2001;97(9):2798-807.
17. Knittel G, Liedgens P, Korovkina D, Seeger JM, Al-Baldawi Y, AI-Maarri M, et al. B-cell-specific conditional expression of Myd88p.L252P leads to the development of diffuse large B-cell lymphoma in mice. Blood. 2016;127(22):2732-41.
18. Prescott JA, Cook SJ. Targeting IKKbeta in Cancer: Challenges and Opportunities for the Therapeutic Utilisation of IKKbeta Inhibitors. Cells. 2018;7(9).
19. Chapuy B. et al. Molecular subtypes of diffuse large B cell lymphoma are associated with distinct pathogenic mechanisms and outcomes. Nat Med. 2018 May;24(5):679-690. doi: 10.1038/s41591-018-0016-8.

## Claims

1. **A compound for use in the treatment and/or prevention of a proliferative disease in a subject,** wherein the compound is selected from:
(a) A Receptor-interacting serine/threonine-protein kinase 1 (RIPK1) inhibitor;
(b) An Inhibitor of κB (IκB) Kinase (IKK)/Nuclear Factor κB (NFκB) - signalling inhibitor; and
(c) A B-cell lymphoma 2 (BCL-2) inhibitor,
wherein if the compound is (a), the subject will receive, receives or has received an additional treatment with (b) and (c), or if the compound is (b), the subject will receive, receives or has received an additional treatment with (a) and (c), or if the compound is (c), the subject will receive, receives or has received an additional treatment with (a) and (b).

2. The compound for use according to claim 1, wherein the compound is provided in a therapeutic composition comprising one or two additional compounds selected from (a) to (c), or wherein the compound is provided in a therapeutic composition comprising all of (a) to (c).

3. The compound for use according to claim 1 or 2, wherein the IKK/NFκB signaling inhibitor (b) is an IKK inhibitor, preferably an IKK2/IKKβ inhibitor.

4. The compound for use according to any one of claims 1 to 3, wherein any one of (a) to (c) is selected from a small molecule, a polypeptide, a peptide, a glycoprotein, a peptidomimetic, an antigen binding protein (ABP) (for example, an antibody, antibody-like molecule or other antigen binding derivative, or an antigen-binding fragment thereof), a nucleic acid, such as a DNA or RNA, for example an antisense or inhibitory DNA or RNA, a ribozyme, an RNA or DNA aptamer, RNAi, siRNA, shRNA and the like, including variants or derivatives thereof such as a peptide nucleic acid (PNA), a genetic construct for targeted gene editing, such as a CRISPR/Cas9 construct and/or a guide nucleic acid (gRNA or gDNA) and/or tracrRNA.

5. **A pharmaceutical composition,** the pharmaceutical composition comprising a RIPK1 inhibitor, an IKK/ NFκB - signalling inhibitor, and a BCL-2 inhibitor; and a pharmaceutically active carrier and/or excipient.

6. **A pharmaceutical composition for use in the treatment and/or prevention of a proliferative disease in a subject,** the pharmaceutical composition comprising a RIPK1 inhibitor, an IKK/ NFκB - signalling inhibitor, and a BCL-2 inhibitor; and a pharmaceutically active carrier and/or excipient.

7. **A combination for use in the treatment and/or prevention of a proliferative disease in a subject,** wherein the combination comprises a RIPK1 inhibitor, an IKK/NFκB signaling inhibitor, and a BCL-2 inhibitor.

8. The compound for use according to any one of claims 1 to 4, the pharmaceutical composition according to claim 5, the pharmaceutical composition for use according to claim 6, or the combination for use according to claim 7, wherein the RIPK1 inhibitor is selected from the group consisting of necrostatin-1 stable (5-((7-chloro-1 H-indol-3-yl)methyl)-3-methyl-2,4-imidazolidinedione), necrostatin-1 (5-(1H-indo1-3-ylmethyl)-3-methyl-2-thioxo-4-imidazolidinone), necrostatin-2 (5-[(7-chloro-1H-indol-3-yl)methyl]-3-methyl-2,4-imidazolidinedione), necrostatin-3 (3-phenyl-3,3a,4,5-tetrahydro-2H-benz[g]indazole, 3-phenyl-2,3,3a,4-tetrahydro[1]benzopyrano[4,3-c]pyrazole, 3-phenyl-2,3,3a,4-tetrahydro[1]benzothiopyrano[4,3-c]pyrazole, 5,5-dioxo-3-phenyl-2,3,3a,4-tetrahydro[1]benzothiopyrano[4,3-c]pyrazole]), necrostatin-4 ((S)-N-(1-[2-chloro-6-fluorophenyl]ethyl)-5-cyano-1-methyl-1H-pyrrole-2-carboxamide), necrostatin-5 (2-[[3,4,5,6,7,8-hexahydro-3-(4-methoxyphenyl)-4-oxo[1]benzothieno[2,3-d]pyrimidin-2-yl]thio]-acetonitrile), necrostatin-7(5-((3-(4-fluorophenyl)-1H-pyrazol-4-yl)methylene)-2-imino-3-(thiazol-2-yl)thiazolidin-4-one), necrostatin-21, (S)-5-benzyl-N-(7,9difluoro-2-oxo-2,3,4,5-tetrahydro-1H-benzo[b]azepin-3-yl)1H-1,2,4-triazole-3-carboxamide (GSK3145095), N-benzyl-N-hydroxy-2,2-dimethylbutanamide (RIPA-56), DNL747 (SAR 443060), (S)-N-(5-methyl-4-oxo-2,3,4,5-tetrahydrobenzo[b][1,4]oxazepin-3-yl)-4,5,6,7-tetrahydro-1H-indazole-3-carboxamide, (S)-N-(5-methyl-4-oxo-2,3,4,5-tetrahydrobenzo[b][1,4]oxazepin-3-yl)-4H-1,2,4-triazole-3-carboxamide, (R)-5-methyl-N-((S)-5-methyl-4-oxo-2,3,4,5-tetrahydrobenzo[b][1,4]oxazepin-3-yl)-4,5,6,7-tetrahydro-1H-indazole-3-carboxamide, (R)-5-methyl-N-((S)-5-methyl-4-oxo-2,3,4,5-tetrahydrobenzo[b][1,4]oxazepin-3-yl)-4,5,6,7-tetrahydro-1H-indazole-3-carboxamide, (S)-6,6-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (S)-N-(5-methyl-4-oxo-2,3,4,5-tetrahydrobenzo[b][1,4]oxazepin-3-yl)-1',4',5',7'-tetrahydrospiro[cyclopropane-1,6'-indazole]-3'-carboxamide, N-((S)-5-methyl-4-oxo-2,3,4,5-tetrahydrobenzo[b][1,4]oxazepin-3-yl)-1,4,4a,5,5a,6-hexahydrocyclopropa[f]indazole-3-carboxamide, N-((S)-5-methyl-4-oxo-2,3,4,5-tetrahydrobenzo[b][1,4]oxazepin-3-yl)-3,4,5,5a,6,6a-hexahydrocyclopropa[e]indazole-1-carboxamide, (S)-5,5-dimethyl-N-(5-methyl-4-oxo-2,3,4,5-tetrahydrobenzo[b][1,4]oxazepin-3-yl)-4,5,6,7-tetrahydro-1H-indazole-3-carboxamide, (R)-N-((S)-5-methyl-4-oxo-2,3,4,5-tetrahydrobenzo[b][1,4]oxazepin-3-yl)-5- (trifluoromethyl)-4,5,6,7-tetrahydro-1H-indazole-3-carboxamide, (R)-5-(tert-butyl)-N-((S)-5-methyl-4-oxo-2,3,4,5-tetrahydrobenzo[b][1,4]oxazepin-3-yl)-4,5,6,7-tetrahydro-1H-indazole-3-carboxamide, N-((S)-5-methyl-4-oxo-2,3,4,5-tetrahydrobenzo[b][1,4]oxazepin-3-yl)-5-phenyl-4,5,6,7-tetrahydro-1H-indazole-3-carboxamide, N-((S)-5-methyl-4-oxo-2,3,4,5-tetrahydrobenzo[b] [1,4]oxazepin-3-yl)-5-(1H-pyrazol-1-yl)-4,5,6,7-tetrahydro-1H-indazole-3-carboxamide, (R)-1-methyl-N-((S)-5-methyl-4-oxo-2,3,4,5-tetrahydrobenzo[b][1,4]oxazepin-3-yl)-5-(triHuoromethyl)-4,5,6,7-tetrahydro-1H-indazole-3-carboxamide, (S)-N-(5-methyl-4-oxo-2,3,4,5-tetrahydrobenzo[b][1,4]oxazepin-3-yl)-5-(triHuoromethyl)-1H-indazole-3-carboxamide, (S)-N-(5-methyl-4-oxo-2,3,4,5-tetrahydrobenzo[b][1,4]oxazepin-3-yl)-5-(trifluoromethyl)-1H-pyrazolo[3,4-b]pyridine-3-carboxamide, (R)-N-((S)-5-methyl-4-oxo-2,3,4,5-tetrahydrobenzo[b][1,4]oxazepin-3-yl)-5-(trifluoromethyl)-4,5,6,7-tetrahydro-[1,2,3]triazolo [1,5-a]pyridine-3-carboxamide, (S)-N-(5-methyl-4-oxo-2,3,4,5-tetrahydrobenzo[b][1,4]oxazepin-3-yl)-7-(trifluoromethyl)imidazo[1,5-a]pyridine-1-carboxamide, (S)-N-(5-methyl-4-oxo-2,3,4,5-tetrahydrobenzo[b][1,4]oxazepin-3-yl)-6- (trifluoromethyl)-[1,2,4]triazolo[4,3-b]pyridazine-3-carboxamide, (S)-5-isopropyl-N-(5-methyl-4-oxo-2,3,4,5-tetrahydrobenzo[b][1,4]oxazepin-3-yl)-1H-pyrazolo[3,4-b]pyridine-3-carboxamide, (S)-N-(5-methyl-4-oxo-2,3,4,5-tetrahydrobenzo[b][1,4]oxazepin-3-yl)-5-(perfluoroethyl)-1H-pyrazolo[3,4-b]pyridine-3-carboxamide, N-((S)-5-methyl-4-oxo-2,3,4,5-tetrahydrobenzo[b][1,4]oxazepin-3-yl)-5-(perfluoroethyl)-4,5,6,7-tetrahydro-[1,2,3]triazolo[1,5-a]pyridine-3-carboxamide, (S)-N-(5-methyl-4-oxo-2,3,4,5-tetrahydrobenzo[b][1,4]oxazepin-3-yl)-6-(perfluoroethyl)-[1,2,4]triazolo[4,3-b]pyridazine-3-carboxamide, (S)-5-benzyl-N-(5-methyl-4-oxo-2,3,4,5-tetrahydrobenzo[b][1,4]oxazepin-3-yl)isoxazole-3-carboxamide (GSK481), 1-[(5S)-4,5-Dihydro-5-phenyl-1H-pyrazol-1-yl]-2,2-dimethyl-1-propanone, 2,2-Dimethyl-1-(5(S)-phenyl-4,5-dihydro-pyrazol-1-yl)-propan-1-one (GSK963), (S)-5-benzyl-N-(5-methyl-4-oxo-2,3,4,5-tetrahydrobenzo[b][1,4]oxazepin-3-yl)-1H-1,2,4-triazole-3-carboxamide (GSK2982772), 5-(indol-3-ylmethyl)-3-methyl-2-thio-hydantoin), and their derivatives or analogues; and preferably is necrostatin-1 stable (Nec-is).

9. The compound for use according to any one of claims 1 to 4, or 8, the pharmaceutical composition according to claim 5 or 8, the pharmaceutical composition for use according to claim 6 or 8, or the combination for use according to claim 7 or 8, wherein the IKK/NFκB signaling inhibitor is an IKK inhibitor, preferably an IKK2/IKKβ inhibitor, more preferably wherein the IKK2/IKKβ inhibitor is selected from the group consisting of N-(6-chloro-7-methoxy-9H-beta-carbolin-8-yl)-2-methylnicotinamide (ML120B), LY2409881, (3S)-N-(6-Chloro-9H-beta-carbolin-8-yl)-4-(2-((2R,6S)-2,6-dimethylmorpholin-4-yl)-2-oxoethyl)-6,6-dimethylmorpholine-3-carboxamide (MLN-0415), 2-(pyrimidin-4-yl)-1H-indole (SAR-113945), 1-(5-Methoxy-2-thiophen-2-yl-quinazolin-4-ylamino)-3-methyl-pyrrole-2,5-dione (CDC-839), 1H-imidazo[4,5-b]pyridin-5-amine, 7-[5-[(cyclohexylmethylamino)-methyl]-1H-indol-2-yl]-2-methyl, sulfate trihydrate (E-6070), TPCA-1, Bardoxolone Methyl, Resveratrol, MRT67307, AmLexanox, BMS-345541, IKK 16, BI605906, ACHP Hydrochloride, PS-1145, Apigenin, SC-514, IKKε-IN-1, IMD-0354, AZD 3264, Icariin (Ieariline), Bay 65-1942 Hcl, Bay 65-1942, an Anilino-Pyrimidine, IKK inhibitor III, SC-514*Amino-imidazolecarboxamide, an Ureudo-thiophenecarboxamide, a Diarylpybidine, a Pyridooxazinone, an Indolecarboxamide, a Benzoimidazole carboxamide, a Pyrazolo[4,3-c]quinoline, an Imidazolylquinoline-carbxaldehyde semicarbazide, a Pyridyl Cyanoguanidine, IkB Kinase Inhibitor Peptide, IKK-2 Inhibitor IV ([5-(p- Fluorophenyl)-2-ureido]thiophene-3-carboxamide), IKK Inhibitor II, Wedelolactone, IKK Inhibitor VII, IKK-2 Inhibitor V (N-(3,5-Bis-trifluoromethylphenyl)-5-chloro-2-hydroxybenzamide), IKK-2 Inhibitor VI (5- Phenyl-2-ureido)thiophene-3-carboxamide), IKK-2 Inhibitor VIII ACHP (2-Amino-6-(2-(cyclopropylmethoxy)-6-hydroxyphenyl)-4-(4-piperidinyl)-3-pyridinecarbonitrile), and their derivatives or analogues, and preferably is LY2409881.

10. The compound for use according to any one of claims 1 to 4, or 8 or 9, the pharmaceutical composition according to claim 5, 8 or 9, the pharmaceutical composition for use according to claim 6, 8 or 9, or the combination for use according to any one of claims 7 to 9, wherein the BCL-2 inhibitor is selected from the group consisting of 4-[4-[[2-(4-chlorophenyl)-5,5-dimethyl-1-cyclohexen-1-yl]methyl]-1-piperazinyl]-N-[[4-[[(1R)-3-(4-morpholinyl)-1-[(phenylthio)methyl]propyl]amino]-3-[(trifluoromethyl)sulfo nyl]phenyl] sulfonyl]benzamide (navitoclax or ABT-263), tetrocarcin A, antimycin, gossypol (such as (-)-gossypol acetic acid), obatoclax (GX15-070), ethyl 2-amino-6-bromo-4-(1-cyano-2-ethoxy-2-oxoethyl)-4H-chromene-3-carboxylate (HA 14-1), oblimersen, a Bak BH3 peptide, 4-[4-[(4'-chloro[1,1'-biphenyl]-2-yl)methyl]-1-piperazinyl]-N-[[4-[[(1R)-3-(dimethylamino)-1-[(phenylthio)methyl]propyl]amino]-3-nitrophenyl]sulfonyl]-benzamide (ABT-737), 2-((1H-pyrrolo[2,3-b]pyridin-5-yl)oxy)-4-(44(4'-chloro-5,5-dimethyl-3,4,5,6-tetrahydro-[1,1'-biphenyl]-2-Amethyl)piperazin-1-yl)-N-((3-nitro-4-(((tetrahydro-2H-pyran-4-Amethyl)amino)phenyl)sulfonyl)benzamide (venetoclax), S55746 (BCL201), and their derivatives or analogues; and preferably is venetoclax.

11. The compound for use according to any one of claims 1 to 4, or any one of claims 8 to 10, the pharmaceutical composition according to claim 5, or any one of claims 8 to 10, the pharmaceutical composition for use according to claim 6, or any one of claims 8 to 10, or the combination for use according to any one of claims 7 to 10, wherein the RIPK1 inhibitor is Nec-is, the IKK/ NFκB - signalling inhibitor is LY2409881, and the BCL-2 inhibitor is venetoclax, or any derivative, analogue, or salt of these compounds.

12. The compound for use according to any one of claims 1 to 4, or any one of claims 8 to 11, the pharmaceutical composition according to claim 5, or any one of claims 8 to 11, the pharmaceutical composition for use according to claim 6, or any one of claims 8 to 11, or the combination for use according to any one of claims 7 to 11, wherein the proliferative disorder is preferably a solid cancer or a hematologic cancer, for example including leukemias, lymphomas, and myelomas.

13. The compound for use according to any one of claims 1 to 4, or any one of claims 8 to 12, the pharmaceutical composition according to claim 5, or any one of claims 8 to 12, the pharmaceutical composition for use according to claim 6, or any one of claims 8 to 12, or the combination for use according to any one of claims 7 to 12, wherein the proliferative disease is a cancer selected from B cell non-Hodgkin's lymphoma (B-NHL), T cell non-Hodgkin's lymphoma (T-NHL), acute myelogenous leukemia (AML), acute lymphoblastic leukemia (ALL), such as B-cell ALL or T-cell ALL, for example precursor B-cell ALL, precursor T-cell ALL, mature B-cell ALL, or mature T-cell ALL, chronic lymphocytic leukemia (CLL), small lymphocytic lymphoma (SLL), Hodgkin's lymphoma, multiple myeloma, myelodysplastic syndrome (MDS), mantle-cell lymphoma (MCL), diffuse large B-cell lymphoma, activated B cell type diffuse large B cell lymphoma (ABC-DLBCL), cluster 1 diffuse large B cell lymphoma, and cluster 5 diffuse large B cell lymphoma; and preferably is B cell non-Hodgkin's lymphoma (B-NHL) and/or B-cell mature acute lymphoblastic leukemia (B-cell ALL).

14. The compound for use according to any one of claims 1 to 4, or any one of claims 8 to 13, the pharmaceutical composition according to claim 5, or any one of claims 8 to 13, the pharmaceutical composition for use according to claim 6, or any one of claims 8 to 13, or the combination for use according to any one of claims 7 to 13, wherein the subject is a mammal, such as a mouse, rat, guinea pig, rabbit, cat, dog, monkey, or preferably a human, for example a human patient, more preferably a human patient suffering from a proliferative disease.

15. **A kit comprising:**
i) a compound for use according to any one of claims 1 to 4, or any one of claims 8 to 14, the pharmaceutical composition according to claim 5, or any one of claims 8 to 14, the pharmaceutical composition for use according to claim 6, or any one of claims 8 to 14, or the combination for use according to any one of claims 7 to 14,
ii) written instructions to apply said compound for use, said pharmaceutical composition, said pharmaceutical composition for use, or said combination for use to a subject; and
iii) optionally, a container holding said compound, said pharmaceutical composition, or said combination for use and the written instructions.
